# EUROPÄISCHE PATENTSCHRIFT

(11) **EP 2 782 666 B1**
(45) Veröffentlichungstag und Bekanntmachung des Hinweises auf die Patenterteilung: **03.02.2016**
(21) Anmeldenummer: 12784290.4
(22) Anmeldetag: 15.11.2012
(51) Int. Cl.: B01F 17/00, C09K 8/68, C07C 41/03, C07C 43/23, C07C 51/41, C07C 59/68, C07C 67/31, C07C 69/734, C09K 8/584, C11D 1/72

(54) **DERIVATE VON TRIS(2-HYDROXYPHENYL)METHANEN, DEREN HERSTELLUNG UND VERWENDUNG FÜR DIE ERDÖLFÖRDERUNG**
DERIVATIVES OF TRIS(2-HYDROXYPHENYL)METHANES, THE PRODUCTION THEREOF AND USE THEREOF FOR OIL PRODUCTION
DÉRIVÉS DE TRIS(2-HYDROXYPHÉNYL)MÉTHANES, LEUR PRODUCTION ET LEUR UTILISATION POUR L'EXTRACTION DE PÉTROLE

(30) Priorität: 24.11.2011 EP 11190566
(43) Veröffentlichungstag der Anmeldung: 01.10.2014
(73) Patentinhaber: Wintershall Holding GmbH, 34119 Kassel (DE)
(72) Erfinder: HANSCH, Markus, 67346 Speyer (DE); MAITRO-VOGEL, Sophie, 68199 Mannheim (DE); RAETHER, Roman, Benedikt, 67346 Speyer (DE)
(74) Vertreter: Poganiuch, Peter
(86) Internationale Anmeldenummer: PCT/EP2012/072758
(87) Internationale Veröffentlichungsnummer: WO 2013/076006

(56) Entgegenhaltungen:
- WO-A1-2008/100436
- SHVETS V ET AL: "Stabilization of Finely Dispersed Suspensions of Water-Insoluble Organic Dyes by Nonionic Surfactants", JOURNAL OF APPLIED CHEMISTRY OF USSR, CONSULTANTS BUREAU, NEW YORK, NY, US, Bd. 58, Nr. 6, 1. Januar 1985 (1985-01-01) , Seiten 1220-1224, XP009116575, ISSN: 0021-888X

## Beschreibung

Die vorliegende Erfindung betrifft neuartige Derivate von Tris(2-hydroxyphenyl)methanen, welche als funktionelle Gruppen Polyalkoxygruppen oder mit terminalensauren Gruppen oder Resten mit 2 OH-Gruppen oder Aminoxidgruppen modifizierte Polyalkoxygruppen aufweisen. Sie betrifft weiterhin die Herstellung derartiger Verbindungen sowie deren Verwendung, insbesondere für die Erdölförderung.

Tris(2-hydroxyphenyl)methane sowie verschiedene Derivate davon sind prinzipiell bekannt.

G. Casiraghi, G. Casnati und M. Cornia, Tetrahedron Letters, No. 9, 679 - 682 (1973) beschreiben die Synthese von mono- oder dialkylierten Tris-(2-hydroxyphenyl)-methanen durch Umsetzung entsprechender Phenole mit Triethylorthoformaten.

M. B. Dinger und M. J. Scott beschreiben in Chem. Commun., 1999, 2525/2526, Inorg. Chem. 2000, 39, 1238 - 1254 sowie Inorg. Chem. 2001, 40, 1029 - 1036 die Synthese verschiedener Tris-(3,5-dialkyl-2-hydroxyphenyl)-methane, wobei als Alkylreste Methyl-, t-Butyl- und t-Pentylreste beschrieben werden. Die Trishydroxyverbindungen werden als Komplexbildner für Zink und Alkalimetallionen verwendet.

M. B. Dinger und M. J. Scott, Eur J. Org. Chem. 2000, 2467 - 2478 , beschreiben weiterhin die weitere Umsetzung der OH-Gruppe von Tris-(3,5-dialkyl-2-hydroxyphenyl)-methanen. Die OH-Funktionen können durch Umsetzen mit Halogencarbonsäureestern und Hydrolyse und/oder weiteren Umsetzungen derivatisiert werden. Dinger und Scott beschreiben beispielsweise Tris(3,5-di-t-butyl-2-carboxymethoxyphenyl)methan, Tris(3,5-di-tert-butyl-2-[(dimethylamido)methoxy]phenyl)methan, Tris{3 ,5-d i-tert-butyl-2-[N-(methylglycyl)carbonylmethoxy]phenyl}methan und Tris(3,5-ditert-butyl-2-[(benzylamino-carbonyl)methoxy]phenyl)-methan. Die Derivate können jeweils als Komplexbildner eingesetzt werden, beispielsweise für Zn(II)-Ionen.

K. Matloka, A. Gelis, M. Regalbuto, G. Vandegift und M. J. Scott, Dalton Trans., 2005, 3719 - 3721 bzw. Separation Science and Technology, 41, 2006, 2129 - 2146 sowie M. W. Peters, E. J. Werner und M. J. Scott, Inorg. Chem., 2002, 41, 1701 - 1716 offenbaren funktionalisierte Tris-(3,5-dialkyl-2-hydroxyphenyl)-methane, und zwar tripodale Diglykolamide und deren Verwendung zur Komplexierung und Abtrennung von Lanthaniden. Als Zwischenstufe der Synthese werden Tris-(3,5-dialkyl-2-hydroxyphenyl)-methane verwendet, bei denen die OH-Gruppe mit ω-Amino- bzw. Cyanoalkylgruppen verethert ist.

R. Mitra, M.W. Peters und M. Scott, Dalton Trans., 2007, 3924 - 3935 , beschreiben weiterhin Tris-(2-hydroxyphenyl)-methan-Derivate, welche terminale 2-Pyridylmethylpiperazin-Gruppen aufweisen. Diese Moleküle können Zinkionen binden und werden als Katalysatoren zur Phosphatdiester- Synthese verwendet. Als Zwischenstufe der mehrstufigen Synthese wird Tris-[2-(2-hydroxylethoxy)-3-methyl-5-t-butylphenyl]methan offenbart.

EP 597 806 A1 offenbart Cyclohexylgruppen-haltige Glydidylether zur Verwendung als Reaktivverdünner, Flexibilisatoren oder Adhäsionsverbesserer. Als Zwischenprodukt der Synthese werden verschiedene Tris(2-hydroxyphenyl)methane beschrieben, darunter auch solche, bei denen die OH-Funktion mit einer (substituierten) 2-Hydroxyethylgruppe verethert ist.

US 2009/0155714 A1 offenbart Zusammensetzungen zur Herstellung von Photoresists. Als Komponente hierfür werden verschiedene Tris(2-hydroxyphenyl)methan-Derivate verwendet, bei denen die OH-Funktion jeweils mit verschiedenen Carbonsäuren ver-estert ist.

Es ist bekannt, dass Tenside oberhalb der kritischen Mizellbildungskonzentration (cmc) zu Mizellen aggregieren. Die Form dieser wasserlöslichen Aggregate hängt von der Struktur der Tenside sowie von äußeren Parametern wie Temperatur oder Elektrolytkonzentration ab. Typischerweise können sich oberhalb der Mizellbildungskonzentration sphärische oder stäbchenförmige Mizellen bilden.

Bei bestimmten strukturellen Gegebenheiten und/oder äußeren Parametern können sich auch lange fadenförmige oder wurmartige Mizellen oder Assoziate bilden. Als Folge davon kommt es bereits bei relativ niedriger Tensidkonzentration zu einer Verschlaufung und Überlappung dieser langen Aggregate, wodurch die Viskosität der Tensidlösung deutlich ansteigt. Eine bestimmte zeitliche Mindeststabilität der Mizellen ist dabei Voraussetzung. Dieses temporär gebildete Netzwerk aus Tensidmizellen reagiert aus rheologischer Sicht sowohl viskos als auch elastisch, weshalb man allgemein von viskoelastischen Tensidlösungen spricht. Mizellen setzen einzelne Tenside frei, nehmen Tenside in den Mizellverband auf, zerfallen und bilden sich wieder neu. Tensidmizellen, die viskoelastische Netzwerke ausbilden, sind zeitlich sehr stabil, bevor sie in einzelne Bruchstücke zerfallen und sich wieder neu bilden, so dass das mizellare Netzwerk einer Scherung der Tensidlösung Widerstand entgegensetzen kann und damit sowohl viskos als auch elastisch reagiert. Weitere Einzelheiten zu viskoelastischen, wurmförmige Mizellen bildenden Tensiden wie Hexadecyltrimethylammonium-p-toluolsulfonat oder Cetyl-pyridinium-salicylat sind beispielweise in H. Hoffmann et al., Adv. Colloid Interface Sci. 1982, 17, 275-298 oder M. R. Rojas et al., Journal of Colloid and Interface Science 342 (2010) 103-109) beschrieben.

Aufgrund der dargestellten Eigenschaften eignen sich viskoleastische Tenside ganz besonders als Verdicker und können in verschiedenen Bereichen der Technik eingesetzt werden.

US 2005/0155762 offenbart Betaine mit Alkylketten von 14 bis 24 C-Atomen, beispielsweise Oleylamidopropylbetain oder Erucylamidorpopylbetain als verdickend wirkende, viskoelastische Tenside.

US 7,461,694 B2 offenbart zwitterionische Tenside mit Alkylketten von 16 bis 24 C-Atomen als viskoelastische Tenside.

WO 2008/100436 A1 offenbart eine viskoelastische Tensidmischung aus kationischen, anionischen oder zwitterionischen Tensiden und einem Polymer. Die Tenside weisen Alkylkettenlängen von 12 bis 25 C-Atomen auf.

In den zitierten Offenbarungen werden zur Bildung viskoelastischer Tensidlösungen jeweils Tenside mit langen Alkylketten eingesetzt. Ein Nachteil von viskoelastischen Tensiden mit langen Alkylketten ist, dass sie bei Kontakt mit unpolaren Flüssigkeiten diese solubilisieren, wodurch die wurmartigen Mizellen in sphärische Aggregate umgewandelt werden und die Viskoelastizität verloren geht. Außerdem bilden diese viskoelastischen Tenside in Kontakt mit anderen Tensiden in der Regel Mischmizellen, wodurch die Viskoelastizität ebenfalls verloren gehen kann. Strukturen mit kurzen Alkylketten oder Strukturen, die vom üblichen linearen Bauprinzip der Tenside abweichen, bilden in der Regel sphärische Mizellen oder lediglich kurze anisometrische Aggregate und somit keine viskoelastischen Tensidlösungen.

Die ältere Anmeldung EP 10163371.7 offenbart Derivate von Tris(2-hydroxyphenyl)methanen, welche gegebenenfalls weiter funktionalisierte terminale Polyalkoxygruppen aufweisen, wobei die Polyalkoxygruppen auch verzweigt sein können. Als Alkoxygruppen werden beispielsweise Ethylenoxygruppen oder C₁- bis C₆-Alkylenoxygruppen verwendet. Die Derivate eignen sich für die Herstellung viskoselastischer Tensidlösungen.

Die ältere Anmeldung EP 11185626.6 offenbart die Verwendung der genannten Derivate von Tris(2-hydroxyphenyl)methanen für die tertiäre Erdölförderung.

Aufgabe der Erfindung war es, neuartige Derivate von Tris(2-hydroxyphenyl)methanen zu finden, welche insbesondere zur Bildung viskoelastischer Tensidlösungen geeignet sein sollten.

Dementsprechend wurden Derivate von Tris(2-hydroxyphenyl)methan gefunden, wobei die Tris(2-hydroxyphenyl)methan-Derivate die allgemeinen Formel (I) 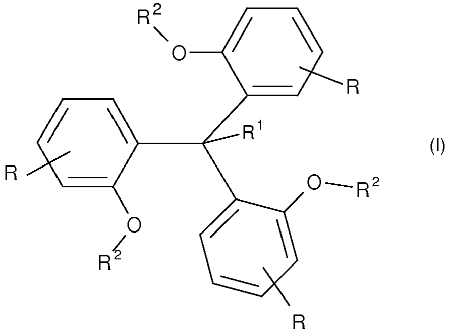 aufweisen, und die Reste R¹, R² und R die nachfolgende Bedeutung haben:
- R:: unabhängig voneinander 0 bis 4 C₁- bis C₃₀-Kohlenwasserstoffreste pro Phenylring,
- R¹ :: ein Rest ausgewählt aus der Gruppe von H, OH, F, Cl, Br, I sowie C₁- bis C₃₀-Kohlenwasserstoffgruppen,
- R²:: unabhängig voneinander Reste der allgemeinen Formel -(-R⁵-O-)ₙ-R⁶-X (III), wobei wobei R⁵, R⁶, X, m und n unabhängig voneinander die folgende Bedeutung haben:
n: eine Zahl von 1 bis 50,
R⁵: unabhängig voneinander Gruppen der allgemeinen Formel -CH₂-CH(R⁷)-(-CH₂)ₘ- (VI), wobei m für 0 oder 1 und R⁷ für einen Rest ausgewählt aus der Gruppe von H, C₁- bis C₆-Kohlenwasserstoffgruppen sowie sauerstoffhaltigen funktionellen Gruppen steht,
R⁶ : eine Einfachbindung oder eine Alkylengruppe mit 1 bis 10 Kohlenstoffatomen, welche optional funktionelle Gruppen als Substituenten aufweisen kann,
X : H oder eine saure Gruppe oder ein Rest mit mindestens 2 OH-Gruppen oder eine Aminoxidgruppe, wobei die Verbindung (I) mindestens einen Rest R⁵ der allgemeinen Formel -CH₂-CH(R^{7a})- (IVa) umfasst, wobei R^{7a} für eine Gruppe ausgewählt aus der Gruppe von - COOR⁸ und -CH₂-O-(-CH₂-CH(R⁹)-O-)_{z}-R¹⁰ steht und R⁸, R⁹, R¹⁰ und z die folgende Bedeutung haben:
R⁸: H, ein a-wertiges Ion der allgemeinen Formel ¹/ₐ M^{a+}, wobei a = 1, 2 oder 3 oder eine Kohlenwasserstoffgruppe mit 1 bis 6 Kohlenstoffatomen,
R⁹: H oder eine Kohlenwasserstoffgruppe mit 1 bis 6 Kohlenstoffatomen,
R¹⁰: H oder eine Kohlenwasserstoffgruppe mit 1 bis 6 Kohlenstoffatomen,
z: eine Zahl von 1 bis 20. Weiterhin wurde die Herstellung derartiger Verbindungen sowie deren Verwendung, insbesondere als Tenside, Verdicker und für die Erdölförderung gefunden.

Zu der Erfindung ist im Einzelnen das Folgende auszuführen:

### Erfindungsgemäße Verbindungen

Bei den erfindungsgemäßen Verbindungen handelt es sich um Derivate von Tris(2-hydroxyphenyl)methan der allgemeinen Formel (I).

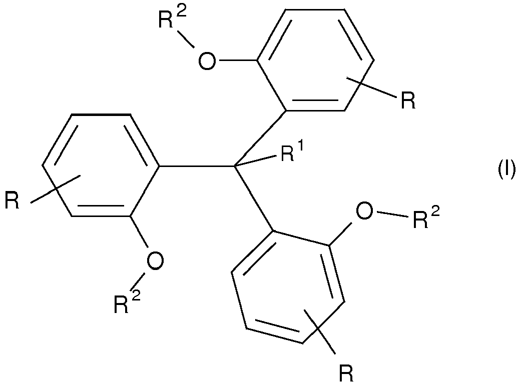

Bei dem Rest R¹ handelt es sich um einen Rest ausgewählt aus der Gruppe von H, OH, F, Cl, Br, I, oder geradkettigen, verzweigten oder cyclischen, aliphatischen und/oder aromatischen C₁-bis C₃₀-Kohlenwasserstoffgruppen. Bevorzugt handelt es sich um H, Cl, eine geradkettige oder verzweigte C₁- bis C₁₂-Alkylgruppe oder eine Benzylgruppe. Besonders bevorzugt handelt es sich bei R¹ um H.

Die drei Phenylringe können jeweils in 3, 4, 5 und 6-Stellung unabhängig voneinander mit Kohlenwasserstoffresten R mit 1 bis 30 Kohlenstoffatomen substituiert sein, wobei die Gruppen beliebig angeordnet sein können. Bevorzugt handelt es sich um 1 oder 2 Gruppen R pro Phenylring. Bei den Gruppen R kann es sich um geradkettige, verzweigte oder cyclische, aliphatische und/oder aromatische Kohlenwasserstoffreste handeln. Bevorzugt handelt es sich bevorzugt um geradkettigte, verzweigte oder cyclische aliphatische Kohlenwasserstoffgruppen mit 1 bis 20, besonders bevorzugt 1 bis 12 Kohlenstoffatomen. Beispiele geeigneter Gruppen R umfassen Methyl-, Ethyl-, n-Propyl-, Isopropyl-, n-Butyl-, i-Propyl-, t-Butyl, n-Pentyl-, 1,1-Dimethylpropyl-, 2,2-Dimethylpropyl, 3-Methylbutyl-, Hexyl-, 2-Ethylhexyl, Heptyl-, Octyl-, Nonyl-, Decyl-, Undecyl-, Dodecyl-, Cyclopentyl-, Cyclohexyl-, Adamantyl- oder Benzylgruppen.

Bevorzugt weisen die erfindungsgemäßen Verbindungen die allgemeine Formel (II) auf.

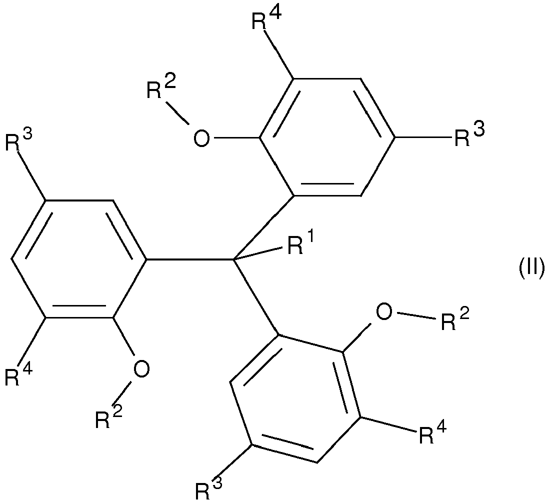

In Formel (II) stehen R³ und R⁴ jeweils unabhängig voneinander für H oder Kohlenwasserstoffreste mit 1 bis 30 C-Atomen, bevorzugt 1 bis 20 C-Atomen, besonders bevorzugt 1 bis 12 C-Atomen. Die Kohlenwasserstoffreste können geradkettig, verzweigt, cyclisch, aliphatisch und/oder aromatisch sein. Bevorzugt handelt es sich um geradkettigte, verzweigte oder cyclische aliphatische Kohlenwasserstoffgruppen mit 1 bis 20, besonders bevorzugt 1 bis 12 Kohlenstoffatomen und ganz besonders bevorzugt um geradkettige oder verzweigte aliphatische Kohlenwasserstoffreste mit 1 bis 6 Kohlenstoffatomen.

Beispiele geeigneter Kohlenwaserstoffgruppen umfassen Methyl-, Ethyl-, Propyl-, Isopropyl-, n-Butyl-, i-Butyl, t-Butyl, n-Pentyl-, 1,1-Dimethylpropyl-, 2,2-Dimethylpropyl, 3-Methylbutyl-, Hexyl-, 2-Ethylhexyl, Heptyl-, Octyl-, Nonyl-, Decyl-, Undecyl-, Dodecyl-, Cyclopentyl-, Cyclohexyl- oder Adamantylgruppen.

Bevorzugt handelt es sich bei R³ und R⁴ um H oder Methyl-, Ethyl-, Propyl-, Isopropyl-, n-Butyl-, i-Butyl, t-Butyl, n-Pentyl-, 1,1-Dimethylpropyl-, 2,2-Dimethylpropyl, 3-Methylbutylgruppen, 1,1,3,3-Tetramethylbutyl, besonders bevorzugt um t-Butylgruppen.

In einer bevorzugten Ausführungsform der Erfindung ist mindestens einer der Reste R³ oder R⁴ nicht H, besonders bevorzugt ist bei dieser Ausführungsform der Rest R³ nicht H. Ganz besonders bevorzugt sind beide Reste R³ und R⁴ jeweils nicht H. Bevorzugte, besonders bevorzugte und ganz besonders bevorzugte Kombinationen von Resten R³ und R⁴ sind in den nachfolgenden Tabellen 1, 2 und 3 genannt:

**Tabelle 1: Liste bevorzugter Kombination**

| R³ | R⁴ |
|---|---|
| t-Butyl | H |
| t-Butyl | Methyl |
| t-Butyl | Ethyl |
| t-Butyl | t-Butyl |
| Me | Me |
| Me | tBu |
| 1,1-Dimethylpropyl | H |
| 1,1-Dimethylpropyl | Methyl |
| 1,1-Dimethylpropyl | Ethyl |
| 1,1-Dimethylpropyl | t-Butyl |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

**Tabelle 2: Liste besonders bevorzugter Kombinationen**

| R³ | R⁴ |
|---|---|
| t-Butyl | Methyl |
| t-Butyl | t-Butyl |
| 1,1-Dimethylpropyl | Methyl |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

**Tabelle 3: Liste ganz besonders bevorzugter Kombinationen**

| R³ | R⁴ |
|---|---|
| t-Butyl | t-Butyl |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

Ganz besonders bevorzugt handelt es sich sowohl bei R³ als auch bei R⁴ um t-Butylreste.

Bei den Resten R² in den oben genannten Formeln (I) und (II) handelt es sich unabhängig voneinander um Reste der allgemeinen Formel -(R⁵-O-)ₙ-R⁶-X (III).

Bei den Resten R⁵ in Formel (III) handelt es sich unabhängig voneinander um Gruppen der allgemeinen Formel -CH₂-CH(R⁷)-(-CH₂)ₘ- (IV).

In der Formel (IV) steht m für 0 oder 1, bevorzugt 0. Es kann sich also um Substituenten R⁷ aufweisende 1,2-Etylengruppen oder 1,3-Propylengruppen handeln.

Bei Gruppen mit m=0 kann der Rest R⁷ anstelle der in Formel (IV) dargestellten Orientierung -CH₂-CH(R⁷)- kann auch in inverser Orientierung -CH(R⁷)-CH₂- in die Polyoxyalkylenkette eingebaut sein. Die Formel (IV) soll beide Orientierungen umfassen, wobei selbstverständlich auch beide Orientierungen in einer Kette enthalten sein können.

Die Reste R⁷ stehen unabhängig voneinander für einen Reste ausgewählt aus der Gruppe von H, C₁- bis C₆-Kohlenwasserstoffgruppen sowie sauerstoffhaltigen funktionellen Gruppen.

Bei den C₁- bis C₆-Kohlenwasserstoffgruppen kann es sich um geradkettige, verzweigte, gesättigte, ungesättigte oder aromatische Kohlenwasserstoffgruppen handeln. Bevorzugt handelt es sich um aliphatische Kohlenwasserstoffgruppen. Beispiele derartiger Gruppen umfassen Methyl-, Ethyl-, n-Propyl oder Phenylreste.

Beispiele sauerstoffhaltiger funktioneller Gruppen umfassen -OH, -CH₂OH, -CH₂OR¹¹, wobei es sich bei R¹¹ um einen Kohlenwasserstoffrest, bevorzugt mit 1 bis 6 Kohlenstoffatomen handelt, Polyalkoxygruppen, welche eine terminale OH-Gruppe umfassen können oder eine weiter funktionalisierte OH-Gruppe. Derartige Gruppen können die allgemeine Formel -(R⁵-O-)ₙ-R⁶-X aufweisen, wobei R⁵, R⁶, X und n jeweils die vorstehend und nachstehend geschilderte Bedeutung haben.

In den obigen Resten steht X jeweils für H oder eine saure Gruppe oder einen Rest mit mindestens 2 OH-Gruppen oder eine Aminoxidgruppe. Bei sauren Gruppen handelt es sich bevorzugt um eine Gruppe ausgewählt aus der Gruppe von Carboxylgruppen -COOM, Sulfonsäuregruppen -SO₃M, Sulfatgruppen -OSO₃M, Phosphonsäuregruppen -PO₂M₂ oder Phoshorsäuregruppen -OPO₃M₂, für wobei M für H⁺ oder ein einwertiges Gegenion steht. Die sauren Gruppen können also als freie Säure vorliegen und/oder als ein Salz davon. Sofern es sich bei M nicht um H⁺ handelt, handelt es sich bevorzugt um ein einwertiges Gegenion, wie beispielsweise NH₄⁺-, Ammoniumionen mit organischen Resten oder Alkalimetallionen. Bevorzugte saure Gruppen sind solche ausgewählt aus der Gruppe von Carboxylgruppen -COOM, Sulfonsäuregruppen -SO₃M oder Sulfatgruppen -OSO₃M, besonders bevorzugt Sulfatgruppen -OSO₃M. Bevorzugte Gruppen mit mindestens 2 OH-Gruppen umfassen insbesondere Mono- oder Oligosaccharidreste, bevorzugt Monosaccharidreste. Es kann sich prinzipiell um alle Arten von Sacchariden handeln. Bevorzugt können von Pentosen und Hexosen, insbesondere von Hexosen abgeleitete Reste eingesetzt werden. Beispiele geeigneter Monosaccharide umfassen Glucose, Mannose, Galaktose, Fruktose oder Ribose. Bevorzugt können von Glukose abgeleitete Rest eingesetzt werden. Es kann sich auch um Derivate der Saccharide handeln, beispielsweise um durch Reduktion oder Oxidation aus den Sacchariden hervorgehende Produkte. Insbesondere kann es sich bei derartigen Derivaten um Zuckersäuren wie beispielsweise Gluconsäure handeln.

Je nach Art des Restes R², können nur eine Gruppe X oder auch mehrere Gruppen X in einem Rest R² enthalten sein.

Bei R⁶ handelt es sich um eine Einfachbindung oder eine Alkylengruppe mit 1 bis 10 Kohlenstoffatomen, bevorzugt 1 bis 3 Kohlenstoffatomen, welche optional funktionelle Gruppen als Substituenten, insbesondere eine OH-Gruppe aufweisen kann. Beispiele derartiger Gruppen umfassen -CH₂-, -CH₂CH₂-, -CH₂-CH₂-CH₂- oder -CH₂-CH(OH)-CH₂-Gruppen.

Die Zahl n steht für eine Zahl von 1 bis 50, bevorzugt 2 bis 40 und besonders bevorzugt 3 bis 30 und beispielsweise 5 bis 20.

Wie oben dargestellt, kann ein Rest R⁷ wiederum Einheiten -R⁵-O- umfassen, d.h. die Polyalkoxygruppen R² müssen nicht zwingend linear sein, sondern können auch verzeigt sein. Die Gesamtzahl aller Gruppen R⁵ in einem Rest R², d.h. der Gruppen R⁵ in der Hauptgruppe als auch der Gruppen R⁵ in eventuell vorhandenen Verzweigungen, soll nachfolgend mit z bezeichnet werden. Für den Fall, dass es sich um eine lineare Gruppe R² handelt, entspricht z der Zahl n. Bevorzugt handelt es sich bei z um eine Zahl von 3 bis 50.

Die Zahlen n und z beziehen sich dabei in bekannter Art und Weise auf den Mittelwert der im Molekül vorhandenen Alkoxygruppen, wobei der Mittelwert selbstverständlich nicht eine natürliche Zahl sein muss, sondern auch eine positive rationale Zahl sein kann.

Erfindungsgemäß umfasst die Verbindung (I) mindestens einen Rest R⁵ der allgemeinen Formel -CH₂-CH(R^{7a})- (IVa), wobei R^{7a} für eine Gruppe ausgewählt aus der Gruppe von -COOR⁸ und -CH₂-O-(-CH₂-CH(R⁹)-O-)_{z}-R¹⁰ steht.

R⁸ steht hierbei für H, ein a-wertiges Ion der allgemeinen Formel ¹/ₐ M^{a+}, wobei a = 1, 2 oder 3 oder eine C₁- bis C₆-Kohlenwasserstoffgruppe.

Bei der Kohlenwasserstoffgruppe handelt es sich bevorzugt eine aliphatische Kohlenwasserstoffgruppe und besonders bevorzugt eine Methyl- oder eine Ethylgruppe. Bei den Ionen M handelt es sich bevorzugt um einwertige Ionen, insbesondere Alkalimetallionen wie beispielsweise Li⁺, Na⁺ oder K⁺-lonen oder um Ammoniumionen der allgemeinen Formel NR₄¹¹, wobei die Reste R¹¹ unabhängig voneinander für H oder einen Kohlenwasserstoffrest, beispielsweise einen Kohlenwasserstoffrest mit 1 bis 6, bevorzugt 1 oder 2 Kohlenstoffatomen stehen. Beispiele für Ammoniumionen umfassen NH₄⁺, N(CH₃)₄⁺ oder N(C₂H₅)₄⁺. Mit anderen Worten gesagt, kann es sich bei der Gruppe -COOR⁸ also in einer Ausführungsform der Erfindung um eine Carboxylgruppe -COOH oder Salz davon handeln, und in einer anderen Ausführungsform kann es sich um eine Estergruppe, bevorzugt eine Methylester- oder Ethylestergruppe handeln. Selbstverständlich können auch beide Arten von Gruppen -COOR⁸ in einem Rest R² vorhanden sein.

Bei R⁹ und R¹⁰ handelt es sich um H oder eine Kohlenwasserstoffgruppe mit 1 bis 6 C-Atomen, wobei bei den Kohlenwasserstoffgruppen Methyl- oder Ethylgruppen bevorzugt sind. Z steht für eine Zahl von 1 bis 20, bevorzugt 2 bis 10 und besonders bevorzugt 2 bis 5.

Es ist bevorzugt, dass mindestens 50 mol % der in einem Rest vorhandenen Gruppen R⁹ für H stehen, bevorzugt mindestens 75 % und ganz besonders bevorzugt steht R⁹ ausschließlich für H.

Die Gruppe R¹⁰ steht bevorzugt für eine Methylgruppe.

Der Fachmann trifft unter den möglichen Gruppen (III) sowie der Reste R¹, R², R³ und R⁴ je nach dem gewünschten Verwendungszeck der Verbindungen eine entsprechende Auswahl.

In einer bevorzugten Ausführungsform der Erfindung umfassen die Verbindungen (I) neben den Gruppen mit den Resten R^{7a} weiterhin Reste -CH₂-CH(R^{7b})- (IVb), wobei R^{7b} aus der Gruppe von H, Methyl und Ethyl ausgewählt werden. In der Regel handelt es sich bei mindestens 50 mol % der in einem Rest vorhandenen Gruppen R^{7b} um H, bevorzugt mindestens 75 % und ganz besonders bevorzugt steht R^{7b} ausschließlich für H.

Die Alkoxygruppen (IVa) mit den Resten R^{7a} und die Alkoxygruppen (IVb) mit den Resten R^{7b} können beliebige Art und Weise angeordnet werden, beispielsweise statistisch, blockweise, alternierend oder mit einem Gradienten. Bevorzugt handelt es sich um eine blockweise Anordnung, wobei die Gruppen (IVb) mit den Resten R^{7a} bevorzugt terminal angeordnet sind.

In einer Ausführungsform der Erfindung handelt es sich bei den Resten R² unabhängig voneinander um Reste der allgemeinen Formel -(-CH₂-CH(R^{7b})-O-)ₐ-(-CH₂CH(-COOR⁸)-O-)_{b}-H (V), wobei die Alkylenoxidblöcke in der genannten Reihenfolge angeordnet sind, R^{7b} und R⁸ die oben definierte Bedeutung haben und a und b jeweils für Zahlen von 1 bis 49 stehen, wobei die Summe a + b 1 bis 50 beträgt. Bevorzugt steht a für 2 bis 30, b für 1 bis 20, mit der Maßgabe, dass a > b ist. Besonders bevorzugt steht a für 5 bis 20 und b für 1 bis 10, mit der Maßgabe, dass a > b ist.

Weiterhin steht in der allgemeinen Formel (V) R⁸ bevorzugt für H oder ein Ion ¹/ₐ M^{a+} wie oben definiert, bevorzugt für H, ein Alkalimetallion oder in Ammoniumion, insbesondere NH₄⁺. Besonders bevorzugt handelt es sich bei den Resten (V) um -(-CH₂-CH₂-O-)ₐ-(-CH₂CH(-COOR⁸)-O-)_{b}-H.

### Herstellung der erfindungsgemäßen Verbindungen

Zur Herstellung der erfindungsgemäßen Verbindungen können zunächst Tris(2-hydroxyphenyl)methan-Verbindungen der allgemeinen Formeln (VI) oder (VII) mit dem gewünschten Substitutionsmuster hinsichtlich R¹, und R bzw. R³ und R⁴ synthetisiert werden.

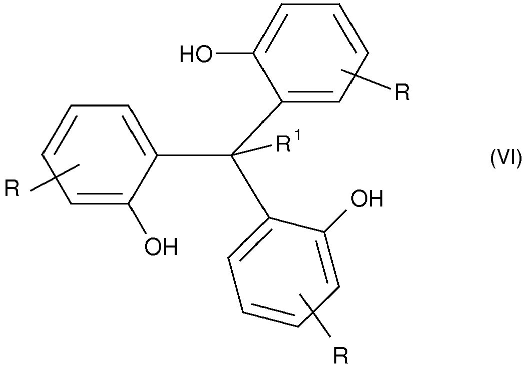

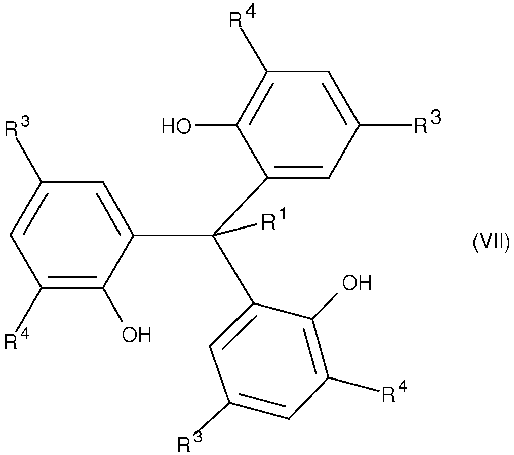

Die Methoden zu Herstellung der Verbindungen sind in der eingangs zitierten Literatur, beispielsweise G. Casiraghi, G. Casnati und M. Cornia, Tetrahedron Letters, No. 9, 679 - 682 (1973), M. B. Dinger und M. J. Scott, Chem. Commun., 1999, 2525/2526, Inorg. Chem. 2000, 39, 1238 - 1254 sowie Inorg. Chem. 2001, 40, 1029 - 1036, M. B. Dinger und M. J. Scott, Eur J. Org. Chem. 2000, 2467 - 2478 , K. Matloka, A. Gelis, M. Regalbuto, G. Vandegift und M. J. Scott, Dalton Trans., 2005, 3719 - 3721, M. W. Peters, E. J. Werner und M. J. Scott, Inorg. Chem., 2002, 41, 1701 - 1716 und R. Mitra, M.W. Peters und M. Scott, Dalton Trans., 2007, 3924 - 3935 ausführlich beschreiben.

Die Tris(2-hydroxyphenyl)methan-Verbindungen der allgemeinen Formeln (VI) oder (VII) können in einem zweiten Schritt in prinzipiell bekannter Art und Weise alkoxyliert werden. Die Durchführung von Alkoxylierungen ist dem Fachmann bekannt. Es ist dem Fachmann ebenfalls bekannt, dass man durch die Reaktionsbedingungen, insbesondere die Wahl des Katalysators, die Molekulargewichtsverteilung der Alkoxylate beeinflussen kann.

Zu Alkoxylierung werden mindestens Alkylenoxide der allgemeinen Formeln 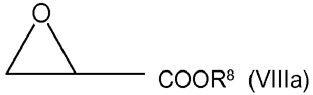 und/oder 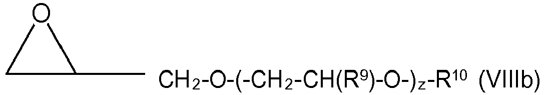 eingesetzt, wobei es sich bei R⁸ im Fall des Alkylenoxids (VIIIa) um einen Kohlenwasserstoffrest mit 1 bis 6 Kohlenwasserstoffatomen handelt, d.h. es werden Ester eingesetzt. Hieraus lassen sich durch nach der Alkoxylierung durch Hydrolyse Carbonsäuregruppen bzw. deren Salze erhalten.

Sollen neben den genannten Alkylenoxiden noch andere Alkylenoxideinheiten in der Resten vorhanden sein, so setzt man neben den Alkylenoxiden (VIIIa) und/oder (Vlllb) noch weitere Alkylenoxide ein, beispielsweise C₂- bis C₈-Alkylenoxide wie Ethylenoxid, Propylenoxid, Butylenoxid oder Styroloxid.

Um Einheiten -CH₂-CH(R⁷)-CH₂- zu erhalten, d.h. m=1, kann man Glycidol (IXa) 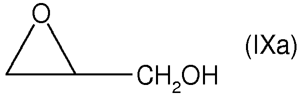 einsetzen. Bei der Alkoxylierung entsteht eine Kette mit einer Endgruppe -CH₂-CH(OH)-CH₂OH, wobei bei fortgesetzter Alkoxylierung an jede der OH-Gruppen weitere Alkylenoxideinheiten angelagert werden können, es werden also verzweigte Gruppen R² erhalten.

Einheiten mit Seitengruppen R⁷ = -CH₂OH können unter Verwendung von geschütztem Glycidol (IXb) erhalten werden.

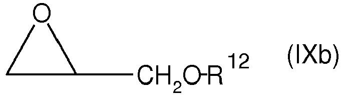

Bei R¹² kann es sich prinzipiell um alle Arten von Gruppen handeln, mit denen sich die OH-Funktion während der Alkoxylierung schützen lassen, beispielsweise t-Butylgruppe oder Benzylgruppen. Die Schutzgruppen können nach der Alkoxylierung oder optional nach Einführung der Gruppen -R⁶-X in prinzipiell bekannter Art und Weise abgespalten werden, wobei Gruppen -CH₂OH entstehen.

Bei der Alkoxylierung kann es sich um eine basenkatalysierte Alkoxylierung handeln. Dazu können die Tris(2-hydroxyphenyl)methan-Verbindungen in einem Druckreaktor mit Alkalimetallhydroxiden, bevorzugt Kaliumhydroxid oder mit Alkalialkoholaten wie beispielsweise Natriummethylat versetzt werden. Durch verminderten Druck (bspw. <100 mbar) und/oder Erhöhung der Temperatur (30 bis 150°C) kann noch in der Mischung vorhandenes Wasser abgezogen werden. Der Alkohol liegt danach als das entsprechende Alkoholat vor. Anschließend wird mit Inertgas (z.B. Stickstoff) inertisiert und das oder die Alkylenoxid(e) bei Temperaturen von 60 bis 180°C bis zu einem Druck von max. 10 bar schrittweise zugegeben. Am Ende der Reaktion kann der Katalysator durch Zugabe von Säure (z.B. Essigsäure oder Phosphorsäure) neutralisiert und kann bei Bedarf abfiltriert werden. Optional kann die Alkoxylierung auch in Gegenwart eines Lösungsmittels durchgeführt werden. Dies kann z.B. Toluol, Xylol, Dimethylformamid oder Ethylencarbonat sein.

Die Alkoxylierung der Alkohole kann aber auch mittels anderer Methoden vorgenommen werden, beispielsweise durch säurekatalysierte Alkoxylierung. Weiterhin können beispielsweise Doppelhydroxidtone wie in DE 43 25 237 A1 beschrieben eingesetzt werden, oder es können Doppelmetallcyanid-Katalysatoren (DMC-Katalysatoren) verwendet werden. Geeignete DMC-Katalysatoren sind beispielsweise in der DE 102 43 361 A1, insbesondere den Abschnitten [0029] bis [0041] sowie der darin zitierten Literatur offenbart. Beispielsweise können Katalysatoren vom Zn-Co-Typ eingesetzt werden. Zur Durchführung der Reaktion kann der Alkohol R-OH mit dem Katalysator versetzt, die Mischung wie oben beschrieben entwässert und mit den Alkylenoxiden wie beschrieben umgesetzt werden. Es werden üblicherweise nicht mehr als 1000 ppm Katalysator bezüglich der Mischung eingesetzt, und der Katalysator kann aufgrund dieser geringen Menge im Produkt verbleiben. Die Katalysatormenge kann in der Regel geringer sein als 1000 ppm, beispielsweise 250 ppm und weniger.
Die Alkoxylierung kann alternativ auch durch Reaktion der Verbindungen (VI) und (VII) mit cyclischen Carbonaten wie beispielsweise Ethylencarbonat vorgenommen werden.

Mittels der Alkoxylierung erhält man bereits erfindungsgemäße Verbindungen, nämlich solche mit X = H. Diese weisen terminale OH-Gruppen auf. Dies ist in der nachfolgenden Abbildung (X) beispielhaft anhand von erfindungsgemäßen Verbindungen gezeigt.

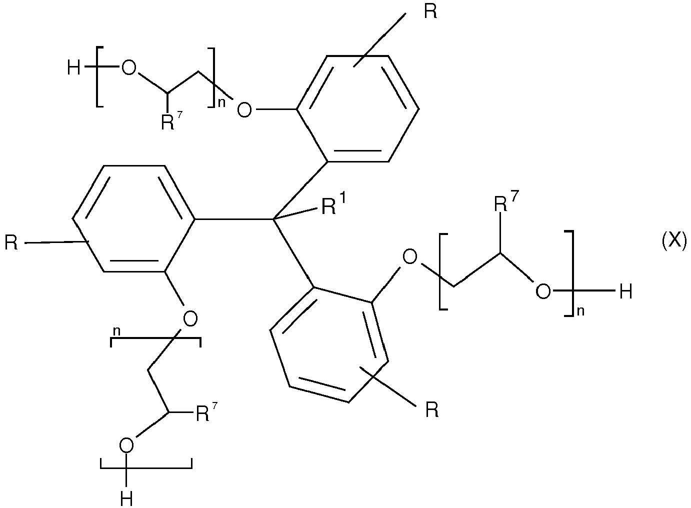

Zur Einführung von Gruppen X, welche nicht H sind, werden die alkoxylierten, terminale OH-Gruppen aufweisenden Tris(2-hydroxyphenyl)methan-Derivate der Formel (X) auf geeignete Art und Weise weiter mit Gruppen -R⁶-X funktionalisiert. Hierdurch werden Verbindungen der allgemeinen Formel (XI) erhalten.

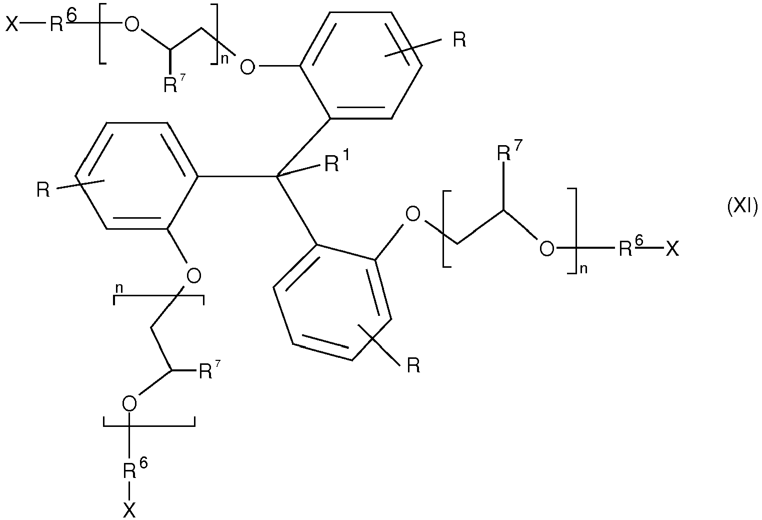

Sulfatgruppen -OSO₃M umfassende Derivate können erhalten werden durch Umsetzung der terminalen OH-Gruppen mit SO₃, Schwefelsäure, Chlorschwefelsäure oder Aminosulfonsäure (CAS-Nr. 5329-14-6) und nachfolgender Neutralisation mit z.B. Natronlauge. Dies kann z.B. in einem Fallfilmreaktor durchgeführt werden. Durch diese Reaktion werden nur die terminalen OH-Gruppen durch Sulfatgruppen substituiert. R⁶ steht bei dieser Reaktion für eine Einfachbindung.

Sulfonatgruppen -SO₃M umfassende Derivate können erhalten werden durch Substitution der OH-Gruppe gegen Cl unter Verwendung von Phosgen oder Thionylchlorid. Die Umsetzung kann in Gegenwart eines Lösungsmittels wie z.B. Chlorbenzol vorgenommen werden. Dabei frei werdende HCl sowie freiwerdendes CO₂ oder SO₂ kann vorteilhaft durch Strippen mit Stickstoff aus dem System entfernt werden, so dass eine Etherspaltung unterbunden wird. Die Alkylalkoxychlor-Verbindung wird im Anschluss daran mit einer wässrigen Lösung von Natriumsulfit umgesetzt, wobei das Chlorid durch Sulfit substituiert wird und das Sulfonat erhalten wird. Die Substitution kann in Gegenwart eines Phasenvermittlers (bspw. C₁- bis C₈-Alkohole) bei einer Temperatur 100 - 180°C und Druck vorgenommen werden. Die Sulfonate können alternativ durch Addition von Vinylsulfonsäure an die Verbindung (V) erhalten werden. Einzelheiten hierzu sind beispielsweise in EP 311 961 A1 beschrieben. Sulfonate können weiterhin erhalten werden, indem man die Verbindungen (V) mit 1,3-Propansulton oder 1,4-Butansulton umsetzt. Hierbei werden Sulfonate mit einer terminalen Gruppe -CH₂-CH₂-CH₂-S0₃M (d.h. R⁶ = CH₂-CH₂-CH₂-) bzw. -CH₂ -CH₂-CH₂-CH₂-SO₃M (d.h. R⁶ = CH₂-CH₂-CH₂-CH₂-) erhalten. Verbindungen mit einer terminalen Gruppe -CH₂-CH(OH)-CH₂-SO₃M (d.h. R⁶ = -CH₂-CH(OH)-CH₂-) lassen sich durch Umsetzung der Verbindung (V) mit Epichlorhydrin und nachfolgender nucleophiler Substitution der Chloridgruppe durch Natriumsulfit erhalten.

Carboxylatgruppen -COOM umfassende Derivate können durch Oxidation der Verbindung (V) erhalten werden. Hierzu sind alle Oxidationsmittel geeignet, gegebenenfalls in Verbindung mit geeigneten Katalysatoren, welche die terminale OH-Gruppe der Verbindung (V) zur COOH-Gruppe oxidieren können, ohne in großem Maße andere Teile des Moleküls zu oxidieren. Die Oxidation kann beispielsweise mit Hilfe von Luft oder Sauerstoff unter Verwendung eines Edelmetallkatalysators (beispielsweise eines Katalysators auf Basis von Palladium) vorgenommen werden. Bei dieser Synthesevariante wird eine terminale Gruppe -CH₂-COOM erhalten (d.h. R⁶ = -CH₂-). Carboxylate können weiterhin auch hergestellt werden, indem man an die OH-Gruppen mittels einer Michael-Addition (Meth)acrylsäure oder einen (Meth)acrylsäureester addiert. Falls die Ester verwendet werden, werden diese nach der Addition verseift. Bei dieser Synthesevariante werden je nachdem, ob Acrylsäure oder (Meth)acrylsäure bzw. deren Ester verwendet wurden- terminale Gruppen -CH₂-CH₂-COOM oder -CH₂-CH(CH₃)-COOM erhalten.

Phosphatgruppen lassen sich durch Umsetzung mit Phosphorpentoxid einführen, Phosphonatgruppen durch Umsetzung mit Vinylphosphonsäure.

Verbindungen mit Mono- oder Oligosaccharidgruppen können hergestellt werden, indem man das entsprechend Saccharid, beispielsweise Glucose mit Hilfe eines sauren Katalysators, wie beispielsweise para-Toluolsulfonsäure und n-Butanol in das entsprechende Butylacetal überführt. Das entstehende Reaktionswasser kann durch Anlegen von Vakuum aus dem Reaktionsgemisch entfernt werden. Im Anschluss wird die Verbindung (V) zugegeben und die Umacetalisierung durch destillative Entfernung des Butanols aus dem Gleichgewicht vorangetrieben. Der saure Katalysator kann am Ende der Reaktion durch Zugabe von Base, beispielsweise NaOH oder KOH neutralisiert werden.

Je nach Art der Gruppen R² weisen die erhaltenden Verbindungen nur eine terminale Gruppe - R⁶-X auf oder auch mehrere terminale und/oder seitenständige Gruppen -R⁶-X.

Bei der Einführung der terminalen Gruppe -R⁶-X müssen selbstverständlich nicht alle OH-Gruppen von OH-terminierten erfindungsgemäßen Verbindungen umgesetzt werden. Es ist möglich, nur einen Teil der Gruppen, beispielsweise nur durchschnittlich jede dritte Gruppe umzusetzen. Auf diese Art und Weise lassen sich die Eigenschaften der erfindungsgemäßen Verbindungen an den gewünschten Verwendungszweck anpassen.

Bei Verwendung von Glycidol sind verschiedene Synthesevarianten denkbar. Verwendet man ungeschütztes Glycidol, so können die Gruppen R² verzweigt sein und mehrere terminale oder seitenständige OH-Gruppen aufweisen. Diese Gruppen können ganz oder auch nur teilweise zur Gruppen -R⁶-X umgesetzt werden. Bei einer nur teilweisen Umsetzung erfolgt die Umsetzung statistisch.

Verwendet man geschütztes Glycidol so entsteht zunächst einmal eine unverzweigte Polyalkoxykette mit einer terminalen OH-Gruppe und seitenständigen, geschützten OH-Gruppen. Man kann nun einerseits die Schutzgruppen zunächst abspalten und dann die Einführung der Gruppen -R⁶-X vornehmen. In diesem Falle entsteht eine lineare Gruppe R², welche end- und/oder seitenständig Gruppen -R⁶-X aufweist. Spaltet man in einer alternativen Synthese die Schutzgruppen zunächst nicht ab, sondern nimmt erst die Einführung der Gruppen -R⁶-X vor, dann reagieren nur die terminalen OH-Gruppen. Die Abspaltung der Schutzgruppen kann danach erfolgen. In diesem Falle entsteht eine Gruppe R², welche eine terminale Gruppe -R⁶-X aufweist und darüber hinaus seitenständige Methylolgruppen -CH₂OH.

Verbindungen (I) mit Gruppen -COOR⁸, bei denen R⁸ für H oder ein Ion steht können erhalten werden, indem man wie beschrieben zunächst eine Verbinddung -COOR⁸ herstellt, bei denen R⁸ für eine Kohlenwasserstoffgruppe steht. Diese kann in einem weiteren Verfahrensschritt in prinzipiell bekannter Art und Weise verseift werden.

### Verwendung der erfindungsgemäßen Verbindungen

Die neuen Verbindungen eignen sich zur Verwendung als Tenside. Sie eignen sich insbesondere zur Herstellung von viskoelastischen Tensidlösungen und können daher als Komponente von verdickenden Formulierungen verwendet werden.

Die neuen Verbindungen eignen sich aufgrund ihrer grenzflächenaktiven Eigenschaften aber auch aufgrund ihrer verdickenden Wirkung weiterhin beispielsweise zum Einsatz in Wasch- und Reinigungsmitteln, Farben und Anstrichmittel, in kosmetischen und pharmazeutischen Formulierungen, Papier-, Textil- und Lederhilfsmitteln, Formulierungen für die Human- und Tierernährung, den Bausektor, Pflanzenschutzformulierungen sowie allgemein zur Herstellung von Emulsionen und Dispersionen.

In einer bevorzugten Ausführungsform der Erfindung können die erfindungsgemäßen Verbindungen in Verfahren für die Erdölförderung verwendet werden, insbesondere zur tertiären Erdölförderung.

Bei der erfindungsgemäßen Verwendung zur Erdölförderung werden in eine Erdöllagerstätte mindestens eine Produktionsbohrung und mindestens eine Injektionsbohrung abgeteuft. In der Regel wird eine Lagerstätte mit mehreren Injektionsbohrungen und mit mehreren Produktionsbohrungen versehen.

Durch die mindestens eine Injektionsbohrung wird eine wässrige Formulierung der beschriebenen Tris(2-hydroxyphenyl)-methan-Derivate (I) in die Erdöllagerstätte injiziert und der Lagerstätte durch mindestens eine Produktionsbohrung Erdöl entnommen. Mit dem Begriff "Erdöl" ist in diesem Zusammenhang nicht nur phasenreines Öl gemeint, sondern der Begriff umfasst auch die üblichen Rohöl-Wasser-Emulsionen. Durch den durch die eingepresste Formulierung erzeugten Druck, fließt das Erdöl in Richtung der Produktionsbohrung und wird über die Produktionsbohrung gefördert.

Die Lagerstätten-Temperatur der Erdöllagerstätte, auf die das erfindungsgemäße Verfahren angewandt wird, beträgt erfindungsgemäß 10 bis 150°C, bevorzugt 10°C bis 120°C und beispielsweise 20°C bis 70°C.

Der Fachmann weiß, dass eine Erdöllagerstätte oftmals, außer bei Thermal-Maßnahmen, eine zeitlich und Areal-bezogen gleichmäßige Temperatur aufweist. Die genannte Lagerstätten-Temperatur bezieht sich auf den Bereich der Lagerstätte zwischen den Injektions- und Produktionsbohrungen, der von der injizierten Zusammensetzung erfasst wird. Methoden zur Ermittlung der Temperatur einer Erdöllagerstätte sind dem Fachmann prinzipiell bekannt. Die Temperatur wird in der Regel aus Temperaturmessungen an bestimmten Stellen der Formation vorgenommen.

Das Verfahren kann insbesondere bei Erdöllagerstätten mit einer durchschnittlichen Permeabilität von 100 mD bis 154 D, bevorzugt 150 mD bis 2 D und besonders bevorzugt 200 mD bis 1 D angewandt werden. Die Permeabilität einer Erdölformation wird vom Fachmann in der Einheit "Darcy" (abgekürzt "D" bzw. "mD" für "Millidarcy") angegeben und kann aus der Fließgeschwindigkeit einer flüssigen Phase in der Erdölformation in Abhängigkeit der angelegten Druckdifferenz bestimmt werden. Die Fließgeschwindigkeit kann in Kernflutversuchen mit der Formation entnommenen Bohrkernen bestimmt werden. Einzelheiten hierzu finden sich beispielsweise in K. Weggen, G. Pusch, H. Rischmüller in "Oil and Gas", Seiten 37 ff., Ulmann's Encyclopedia of Industrial Chemistry, Online-Ausgabe, Wiley-VCH, Weinheim 2010. Für den Fachmann ist klar, dass die Permeabilität in einer Erdöllagerstätte nicht homogen sein muss, sondern im Allgemeinen eine gewisse Verteilung aufweist, und es sich dementsprechend bei der Angabe der Permeabilität einer Erdöllagerstätte um eine durchschnittliche Permeabilität handelt.

Zur erfindungsgemäßen Verwendung wird eine wässrige Formulierung eingesetzt, welche neben Wasser mindestens eines der beschriebenen Tris(2-hydroxyphenyl)-methan-Derivate (I) umfasst. Es können auch Gemische verschiedener Tris(2-hydroxyphenyl)-methan-Derivate eingesetzt werden. Die Formulierung kann in Süßwasser aber auch in Salzen enthaltendem Wasser angesetzt werden. Es kann es sich um Mischungen verschiedener Salze handeln.

Beispielsweise kann Meerwasser zum Ansetzen der wässrigen Formulierung verwendet werden oder es kann gefördertes Formationswasser verwendet werden, welches auf diese Art und Weise wieder verwendet wird. Bei Förderplattformen im Meer wird die Formulierung in der Regel in Meerwasser angesetzt. Bei Fördereinrichtungen an Land kann das Tris(2-hydroxyphenyl)-methan-Derivat vorteilhaft zunächst in Süßwasser gelöst und die erhaltene Lösung mit Formationswasser auf die gewünschte Einsatzkonzentration verdünnt werden. Die Formulierung kann bevorzugt hergestellt werden, indem man das Wasser vorlegt, das Tris(2-hydroxyphenyl)-methan-Derivat als Pulver einstreut und mit dem Wasser vermischt.

Bei den Salzen kann es sich insbesondere um Alkalimetallsalze sowie Erdalkalimetallsalze handeln. Beispiele typischer Kationen umfassen Na⁺, K⁺, Mg²⁺ oder Ca²⁺ und Mg²⁺. Beispiele typischer Anionen umfassen Chlorid, Bromid, Hydrogencarbonat, Sulfat oder Borat.

Sofern die Formulierung Salze umfasst, sind in der Regel zumindest eines oder mehrere Alkalimetallionen, insbesondere zumindest Na⁺ vorhanden. Daneben können auch noch Erdalkalimetallionen vorhanden sein, wobei das Gewichtsverhältnis Alkalimetallionen / Erdalkalimetallionen in der Regel ≥ 2, bevorzugt ≥ 3 ist. Als Anionen sind in der Regel zumindest eines oder mehrere Halogenid-Ionen, insbesondere zumindest Cl⁻ vorhanden. In der Regel beträgt die Menge an Cl⁻ zumindest 50 Gew. %, bevorzugt mindestens 80 Gew. % bezüglich der Summe aller Anionen.

Die Gesamtmenge aller Salze in der wässrigen Formulierung beträgt häufig 10 000 ppm bis 350 000 ppm (Gewichtsanteile), bezüglich der Summe aller Komponenten der Formulierung.

Sofern Meerwasser zum Ansetzen der Formulierung verwendet wird, beträgt der Salzgehalt in der Regel 20 000 ppm bis 50 000 ppm und sofern Formationswasser verwendet wird in der Regel 100 000 ppm bis 250 000 ppm. Die Menge an Erdalkalimetallionen kann bevorzugt 1000 bis 53 000 ppm betragen. Die wässrige Formulierung kann auch weitere Komponenten umfassen, wie z. B. Biozide, Stabilisatoren und Inhibitoren.

Die Konzentration der Tris(2-hydroxyphenyl)-methan-Derivats wird so festgelegt, dass die wässrige Formulierung die gewünschte Viskosität für den Einsatzzweck aufweist. Die Viskosität der Formulierung sollte in der Regel mindestens 3 mPas (gemessen bei 25°C und einer Scherrate von beispielsweise 7 s⁻¹, beziehungsweise wie sie im Reservoir bevorzugt auftritt) betragen, bevorzugt mindestens 10 mPas.

Erfindungsgemäß beträgt die Konzentration der Tris(2-hydroxyphenyl)-methan-Derivate in der Formulierung 0,01 bis 10 Gew.-%, oftmals 0,05 bis 10 Gew.-% bezüglich der Summe aller Komponenten der wässrigen Formulierung. Bevorzugt beträgt die Menge 0,05 bis 5 Gew. %, besonders bevorzugt 0,05 bis 1 Gew. % und beispielsweise ca. 0,1 Gew. %.

Das Injizieren der wässrigen Formulierung kann mittels üblicher Vorrichtungen vorgenommen werden. Die Formulierung kann mittels üblicher Pumpen in eine oder mehrere Injektionsbohrungen injiziert werden. Die Injektionsbohrungen sind oftmals im Bereich der Erdöllagerstätte mit einzementierten Stahlrohren ausgekleidet, und die Stahlrohre sind an der gewünschten Stelle perforiert. Die Formulierung tritt durch die Perforation aus der Injektionsbohrung in die Erdölformation ein. Über den mittels der Pumpen angelegten Druck wird in prinzipiell bekannter Art und Weise die Strömungsgeschwindigkeit der Formulierung und damit auch die Scherbelastung festgelegt, mit der die wässrige Formulierung in die Formation eintritt. Die Scherbelastung beim Eintritt in die Formation kann vom Fachmann in prinzipiell bekannter Art und Weise auf Basis des Gesetz' von Hagen-Poiseuille unter Verwendung der beim Eintritt in die Formation durchströmten Fläche, dem mittleren Porenradius und dem Volumenstrom errechnet werden. Die durchschnittliche Permeabilität bzw. Porosität der Formation kann in prinzipiell bekannter Art und Weise durch Messungen an Bohrkernen ermittelt werden. Die Scherbelastung ist naturgemäß umso größer, je größer der in die Formation injizierte Volumenstrom an wässriger Formulierung ist.

Die Geschwindigkeit der Injektion kann vom Fachmann je nach den Eigenschaften der Formation (Permeabilität, Mächtigkeit) sowie den Erfordernissen des Erdölfeldes (Anzahl der Injektoren, deren Konfiguration, etc.) festgelegt werden.

Bevorzugt beträgt die Scherrate beim Eintritt der wässrigen Formulierung in die Formation mindestens 30000 s⁻¹, bevorzugt mindestens 60000 s⁻¹ und besonders bevorzugt mindestens 90000 s⁻¹.

Häufig eingesetzte Konzentrationen und weitere Komponenten der wässrigen Formulierung:
A) Konzentrationen liegen oft im Bereich von 0,01-10 Gew.-%, oftmals 0,05-10 Gew.-%, bevorzugte Konzentrationen sind zwischen 0,1 - 1 Gew.-% und besonders bevorzugte Konzentrationen zwischen 0,1 und 0,5 Gew.-%, jeweils bezogen auf die gesamte Formulierung.
B) Lösemittel können als weitere Komponente eingesetzt werden. Typischerweise wird das Derivat von Tris-(2-hydroxyphenyl)-methan im Formationswasser gelöst. Auch ein Lösen in Meerwasser ist möglich. Ein Vorlösen mit einem wassermischbaren Lösungsmittel, z. B. Ethanol oder Isopropanol zur Herstellung von Konzentraten mit höherem Aktivgehalt ist möglich. Auch Brunnenwasser kann verwendet werden.
C) Salze haben einen Einfluss auf die Viskosität der Formulierungen. Die Ziel-Viskosität wird oft unter Lagerstätten-Salinität über Variation der Konzentration eingestellt.
D) Eine Abhängigkeit vom pH-Wert der Formulierung auf die verdickenden Eigenschaften bzw. die Viskosität (z. B. bei Einsatz von Carboxylaten) ist möglich.
E) Eine Kombination des Derivats von Tris-(2-hydroxyphenyl)-methan mit einem oder mehreren weiteren Tensiden ist möglich.
F) Optional können auch weitere Komponenten, wie Biozide in der Formulierung eingesetzt werden. In der Regel werden Biozide schon zum Wasserfluten eingesetzt. Insbesondere niedrig-salinare Wässer können mit Algiziden, Fungiziden, etc. behandelt werden.

Die nachfolgenden Beispiele sollen die Erfindung näher illustrieren:

### Herstellung der Ausgangsverbindung:

### Synthese von Tris-(3,5-di-tert-butyl-2-hydroxyphenyl)-methan

Tris-(3,5-di-*tert*-butyl-2-hydroxyphenyl)-methan (CAS-No.143560-44-5) wurde mittels des von M. B. Dinger, M. J. Scott, *Eur. J. Org. Chem.* **2000**, 2467 beschriebenen Verfahrens hergestellt. Tris-(3,5-di-*tert*-butyl-2-hydroxyphenyl)-methan wird nachfolgend auch als TRIS abgekürzt.

### Beispiel 1:

### Synthese von TRIS[(-CH₂-CH₂-O)₉H]₃ durch Ethoxylierung von TRIS mit 27 EO-Einheiten

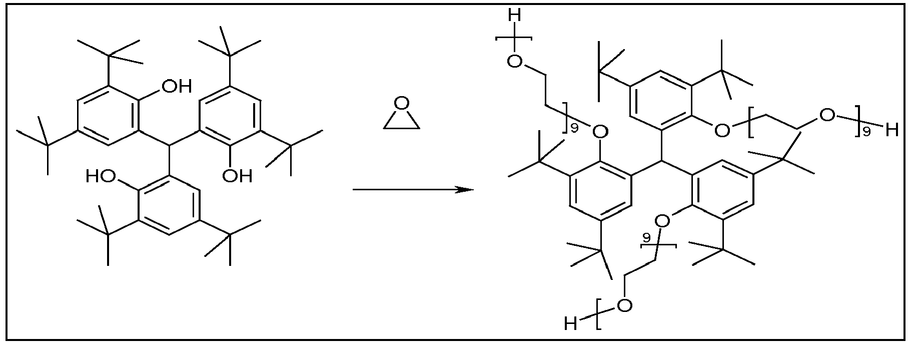

In einem 2 I - Druckreaktor werden 50 g TRIS zusammen mit 3,3 g Kronenether (18-Crown-6) in Toluol gelöst und 1,4 g Kalium-t-butylat zugegeben. Der Versuchsansatz wird gründlich mit Stickstoff gespült, ein Stickstoff-Vordruck von 1,5 bar eingestellt und der Ansatz auf 130 °C erwärmt. Anschließend werden 25 g Ethylenoxid innerhalb von 15 Minuten, dann 70 g Ethylenoxid innerhalb von 90 Minuten zugegeben, wobei eine deutlich exotherme Reaktion eintritt. Der Ansatz wird nach Dosierende 5 Stunden bei 130 °C gerührt, anschließend 12 Stunden bei 50 °C. Danach wird der Ansatz mit Stickstoff gespült und aus dem Reaktor abgelassen. Zu der Reaktionslösung werden 6,6 g Ambersol^{®} gegeben und 2 Stunden bei 80° C und 500 mbar Druck entgast. Anschließend wird die Lösung über einen Tiefenfilter Seitz Supradur^{®} 200 filtriert, das Lösungsmittel vom Filtrat abdestilliert und das Produkt 2 h bei 80° C und 2 mbar getrocknet. Man erhält 144 g Produkt (entspricht 99,3 % der Theorie). Das Produkt entspricht laut 1H-NMR der gewünschten Struktur.

### Beispiel 2:

### Synthese von TRIS[(-CH₂-CH₂-O)₉-(-CH₂-CH(COOCH₃)-O-)₂-H]₃

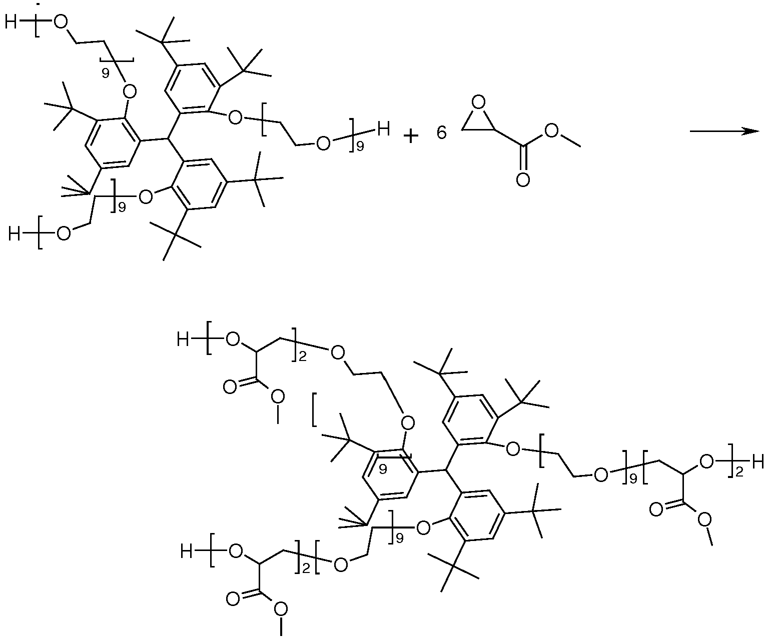

50 g TRIS-27 EO, erhältlich gemäß Beispiel 2, werden zusammen mit 0,1 g Bortrifluorid-Etherat auf 100 °C erwärmt und anschließend 16,7 g Epoxypropionsäuremethylester (EPSMe) zugetropft. Die Reaktion verläuft stark exotherm und der Ansatz wird durch Kühlung im Eisbad zwischen 100 und 150 °C gehalten. Nach vollständiger Zugabe wird der Ansatz weitere 5 h bei 100 °C gerührt. Laut Gaschromatogramm ist kein freier Epoxypropionsäuremethylester (EPSMe) mehr vorhanden. Ausbeute: 65 g (entspricht 98 % der Theorie)

### Beispiel 3:

### Synthese von TRIS[(-CH₂-CH₂-O)₉-(-CH₂-CH(COONa)-O-)₂-H]₃

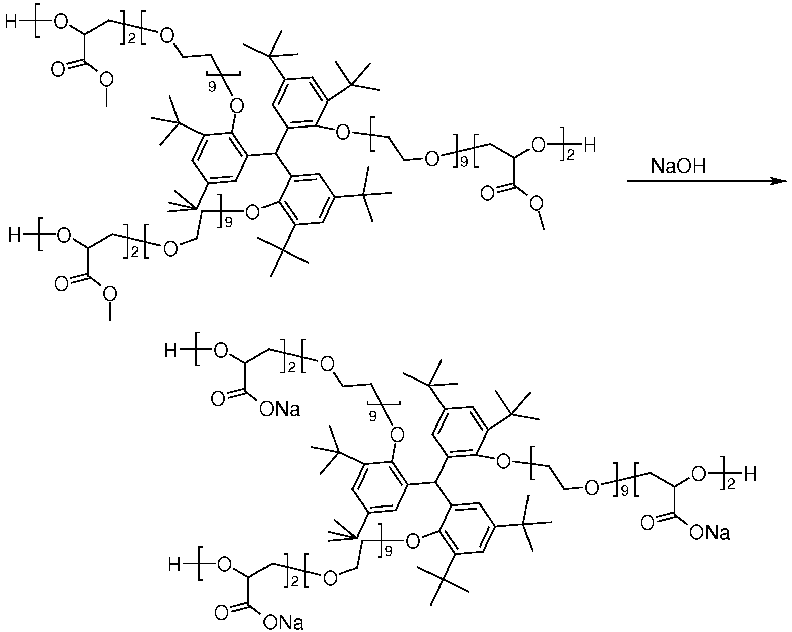

64 g TRIS[(-CH₂-CH₂-O)₉-(-CH₂-CH(COOCH₃)-O-)₂-H]₃, erhältlich gemäß Beispiel 2, werden auf 70 °C erwärmt und innerhalb von 15 Minuten 12, 65 g 50%ige Natriumhydroxid-Lösung zugetropft. Die Reaktion verläuft exotherm und die Reaktionslösung erwärmt sich auf 80 °C. Nach beendeter Zugabe wird der Ansatz weitere 4 h bei 70 °C gerührt. Anschließend werden nacheinander 40 g Wasser, 1 g 50%ige Natriumhydroxid-Lösung und wiederum 30 g Wasser zugegeben. Der pH-Wert der Lösung beträgt zum Reaktionsende 12. Man erhält 140 g einer rotbraunen ca. 50 %igen Emulsion. Das IR-Spektrum belegt die vollständige Verseifung.

## Patentansprüche

1. Derivate von Tris(2-hydroxyphenyl)methanen der allgemeinen Formel (I), 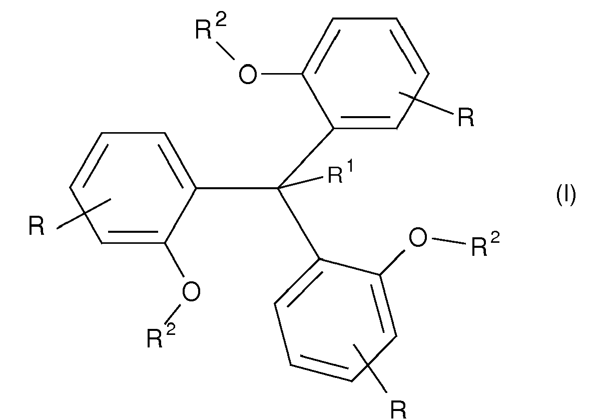 wobei die Reste R¹, R² und R die nachfolgende Bedeutung haben:
R: unabhängig voneinander 0 bis 4 C₁- bis C₃₀-Kohlenwasserstoffreste pro Phenylring,
R¹: ein Rest ausgewählt aus der Gruppe von H, OH, F, Cl, Br, I sowie C₁- bis C₃₀-Kohlenwasserstoffgruppen,
R²: unabhängig voneinander Reste der allgemeinen Formel -(-R⁵-O-)ₙ-R⁶-X (III), wobei wobei R⁵, R⁶, X, m und n unabhängig voneinander die folgende Bedeutung haben:
n: eine Zahl von 1 bis 50,
R⁵: unabhängig voneinander Gruppen der allgemeinen Formel -CH₂-CH(R⁷)-(-CH₂)ₘ- (VI), wobei m für 0 oder 1 und R⁷ für einen Rest ausgewählt aus der Gruppe von H, C₁- bis C₆-Kohlenwasserstoffgruppen sowie sauerstoffhaltigen funktionellen Gruppen steht,
R⁶ : eine Einfachbindung oder eine Alkylengruppe mit 1 bis 10 Kohlenstoffatomen, welche optional funktionelle Gruppen als Substituenten aufweisen kann,
X : H oder eine saure Gruppe oder ein Rest mit mindestens 2 OH-Gruppen oder eine Aminoxidgruppe,
**dadurch gekennzeichnet, dass** die Verbindung (I) mindestens einen Rest R⁵ der allgemeinen Formel -CH₂-CH(R^{7a})- (IVa) umfasst, wobei R^{7a} für eine Gruppe ausgewählt aus der Gruppe von -COOR⁸ und -CH₂-O-(-CH₂-CH(R⁹)-O-)_{z}-R¹⁰ steht und R⁸, R⁹, R¹⁰ und z die folgende Bedeutung haben:
R⁸: H, ein a-wertiges Ion der allgemeinen Formel ¹/ₐ M^{a+}, wobei a = 1, 2 oder 3 oder eine Kohlenwasserstoffgruppe mit 1 bis 6 Kohlenstoffatomen,
R⁹: H oder eine Kohlenwasserstoffgruppe mit 1 bis 6 Kohlenstoffatomen,
R¹⁰: H oder eine Kohlenwasserstoffgruppe mit 1 bis 6 Kohlenstoffatomen,
z: eine Zahl von 1 bis 20.

2. Verbindungen gemäß Anspruch 1, **dadurch gekennzeichnet, dass** es sich bei R^{7a} um eine Gruppe -COOR⁸ handelt und R⁸ für eine Methyl- und/oder Ethylgruppe steht.

3. Verbindungen gemäß Anspruch 1, **dadurch gekennzeichnet, dass** es sich R^{7a} um eine Gruppe -COOR⁸ handelt und R⁸ für H, ein Alkalimetallion oder ein Ammoniumion steht.

4. Verbindungen gemäß Anspruch 1, **dadurch gekennzeichnet, dass** es sich bei R^{7a} um eine Gruppe -CH₂-O-(-CH₂-CH(R⁹)-O-)_{z}-R¹⁰ handelt, wobei mindestens 50 mol % der vorhandenen Reste R⁹ für H stehen, und es sich bei R¹⁰ um eine Methyl- oder eine Ethylgruppe und bei z um eine Zahl von 2 bis 10 handelt.

5. Verbindungen gemäß einem der Ansprüche 1 bis 4, **dadurch gekennzeichnet, dass** Verbindungen (I) neben den Resten (IVa) weiterhin Reste -CH₂-CH(R^{7b})- (IVb) umfassen, wobei R^{7b} aus der Gruppe von H, Methyl und Ethyl ausgewählt wird.

6. Verbindungen gemäß Anspruch 5, **dadurch gekennzeichnet, dass** es sich bei R^{7b} um H handelt.

7. Verbindungen gemäß Anspruch 1, durch gekennzeichnet, dass es sich bei den Resten R² unabhängig voneinander um Reste der allgemeinen Formel -(-CH₂-CH(R^{7b})-O-)ₐ-(-CH₂CH(-COOR⁸)-O-)_{b}-H (V) handelt, wobei die Alkylenoxidblöcke in der genannten Reihenfolge angeordnet sind, R^{7b} und R⁸ die oben definierte Bedeutung haben und a und b jeweils für Zahlen von 1 bis 49 stehen, wobei die Summe a + b 2 bis 50 beträgt.

8. Verbindungen gemäß Anspruch 7, **dadurch gekennzeichnet, dass** in der Formel (V) a für 2 bis 30, b für 1 bis 20, mit der Maßgabe, dass a > b ist.

9. Verbindungen gemäß Anspruch 7 oder 8, **dadurch gekennzeichnet, dass** es sich bei R⁸ um H, ein Alkalimetallion oder ein Ammoniumion handelt.

10. Verbindungen gemäß einem der Ansprüche 1 bis 6 **dadurch gekennzeichnet, dass** die saure Gruppe ausgewählt wird aus der Gruppe von Carboxylgruppen -COOM, Sulfonsäuregruppen -SO₃M, Sulfatgruppen -OSO₃M, Phosphonsäuregruppen -PO₂M₂ oder Phosphorsäuregruppen -OPO₃M₂ handelt, wobei M für H⁺ oder ein einwertiges Gegenion steht.

11. Verbindungen gemäß einem der Ansprüche 1 bis 6 **dadurch gekennzeichnet, dass** der Rest mit mindestens 2 OH-Gruppen ein Mono- oder Oligosaccharidrest ist.

12. Verbindungen gemäß einem der Ansprüche 1 bis 11, **dadurch gekennzeichnet, dass** die Verbindungen die allgemeine Formel (II) 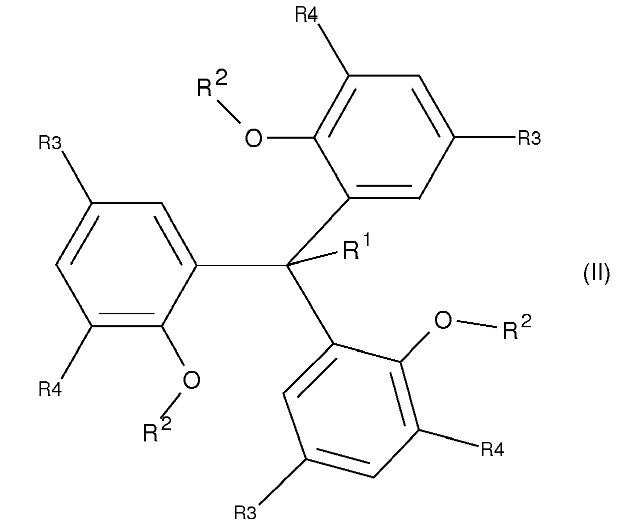 aufweisen, wobei R¹ und R² die oben dargestellte Bedeutung haben und R³ und R⁴ unabhängig voneinander für H oder einen C₁- bis C₃₀-Kohlenwasserstoffrest stehen.

13. Verbindungen gemäß Anspruch 12, **dadurch gekennzeichnet dass** es sich bei R³ und R⁴ unabhängig voneinander um geradkettige oder verzweigte aliphatische C₁- bis C₆- Kohlenwasserstoffreste handelt.

14. Verbindungen gemäß Anspruch 13, **dadurch gekennzeichnet, dass** es sich bei R³ und R⁴ jeweils um t-Butylreste handelt.

15. Verfahren zur Herstellung der Verbindungen gemäß einem der Ansprüche 1 bis 14, **dadurch gekennzeichnet, dass** man eine Ausgangsverbindung der allgemeinen Formel (VI) 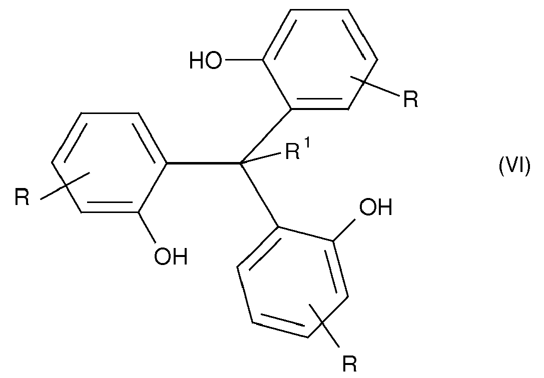 bereitstellt, mit Alkylenoxiden der allgemeinen Formeln 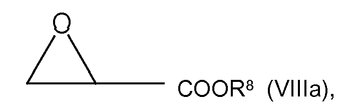 wobei R⁸ für eine Kohlenwasserstoffgruppe mit 1 bis 6 Kohlenstoffatomen steht, und/oder 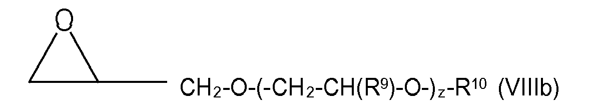 sowie optional davon verschiedenen C₂- bis C₈-Alkylenoxiden, Glycidol und cyclischen C₃- bis C₉-Carbonaten alkoxyliert und hierauf optional terminale H-Atome zumindest teilweise durch Reste -R⁶-X substituiert, wobei z, R, R¹, R⁶, R⁸, R⁹ und R¹⁰ den Definitionen in Anspruch 1 entsprechen.

16. Verfahren gemäß Anspruch 15, **dadurch gekennzeichnet, dass** man Alkylenoxide der Formel (VIIIa) einsetzt und vorhandene COOR⁸-Gruppen nach der Alkoxylierung in einem weiteren Verfahrensschritt zu -COOH-Guppen bzw. Salzen davon hydrolysiert.

17. Verwendung von Verbindungen gemäß einem der Ansprüche 1 bis 14 als Tenside und Verdicker.

18. Verwendung von Verbindungen gemäß einem der Ansprüche 1 bis 14 zur Herstellung von Wasch- und Reinigungsmitteln, Farben und Anstrichmittel, kosmetischen und pharmazeutischen Formulierungen, Papier-, Textil- und Lederhilfsmitteln, Formulierungen für die Human- und Tierernährung, den Bausektor, Pflanzenschutzformulierungen sowie allgemein zur Herstellung von Emulsionen und Dispersionen.

19. Verwendung von Verbindungen gemäß einem der Ansprüche 1 bis 14 in Verfahren für die Erdölförderung.

20. Verwendung gemäß Anspruch 19, indem man eine wässrige Formulierung, die mindestens ein Derivat von Tris-(2-hydroxyphenyl)-methan der allgemeinen Formel (I) enthält, durch mindestens eine Injektionsbohrung in eine Erdöllagerstätte einpresst und der Lagerstätte durch mindestens eine Produktionsbohrung das Rohöl entnimmt.

21. Verwendung gemäß Anspruch 20, **dadurch gekennzeichnet, dass** die Temperatur der Erdöllagerstätte 10 bis 150°C beträgt.

22. Verwendung gemäß Anspruch 19 oder 20, **dadurch gekennzeichnet, dass** die wässrige Formulierung als weitere Komponente mindestens ein Salz in einer Menge von 10.000 ppm bis 350.000 ppm enthält.

23. Verwendung gemäß Anspruch 19, **dadurch gekennzeichnet, dass** die Erdölförderung aus Lagerstätten mit einer Lagerstätten-Temperatur von 10° bis 150°C erfolgt, wobei die Lagerstätte neben Erdöl Lagerstätten-Wasser mit einer Salinität von 20.000 ppm bis 350.000 ppm umfasst und das Erdöl eine Viskosität (gemessen bei Lagerstätten-Temperatur) von mindestens 3 mPa*s aufweist, indem man eine wässrige Formulierung umfassend mindestens ein Derivat von Tris-(2-hydroxyphenyl)-methan der Formel (I) durch mindestens eine Injektionsbohrung in die Erdöllagerstätte einpresst und der Lagerstätte durch mindestens eine Produktionsbohrung Rohöl entnimmt, wobei das Verfahren mindestens die folgenden Verfahrensschritte umfasst:
- Bereitstellen mindestens eines Tris-(2-hydroxyphenyl)-methan-Derivats der allgemeinen Formel (I) als Rein-Substanz, Mischung oder als Konzentrat;
- Herstellen der wässrigen Formulierung der Tris-(2-hydroxyphenyl)-methan-Komponente(n) durch Verdünnen des gemäß Schritt (1) bereitgestellten Konzentrats (K) vor Ort mit Wasser auf eine Konzentration von 0,01 g/l bis 10 g/l, vorzugsweise sowie von 0,05 g/l bis 5 g/l,
- Injizieren der wässrigen Formulierung der Tris-(2-hydroxyphenyl)-methan-Komponente(n) in die Erdölformation, und
- Entnahme von Rohöl durch mindestens eine Produktionsbohrung.

24. Verwendung gemäß einem der Ansprüche 19 bis 23, **dadurch gekennzeichnet, dass** es sich bei dem eingesetzten Derivat von Tris-(2-hydroxyphenyl)-methan um ein Derivat mit unverzweigten Resten R² handelt und die Konzentration des Tris(2-hydroxy-phenyl)methan-Derivats in der Formulierung 0,01 g/l bis 5 g/l beträgt.

## Claims

1. A derivative of tris(2-hydroxyphenyl)methanes of the general formula (I) 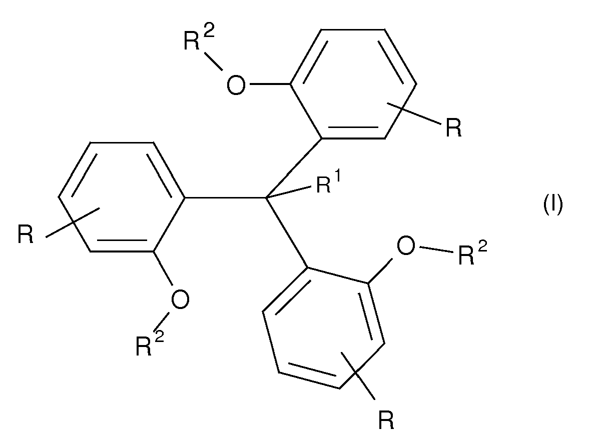 where the R¹, R² and R radicals are each defined as follows:
R: each independently 0 to 4 C₁- to C₃₀-hydrocarbyl radicals per phenyl ring,
R¹: a radical selected from the group of H, OH, F, Cl, Br, I and C₁- to C₃₀-hydrocarbyl groups,
R²: each independently radicals of the general formula -(-R⁵-O-)ₙ-R⁶-X (III), where R⁵, R⁶, X, m and n are each independently defined as follows:
n: a number from 1 to 50,
R⁵: each independently groups of the general formula -CH₂-CH(R⁷)-(-CH₂)ₘ- (VI), where m is 0 or 1 and R⁷ is a radical selected from the group of H, C₁- to C₆-hydrocarbyl groups and oxygen-containing functional groups,
R⁶ : a single bond or an alkylene group which has 1 to 10 carbon atoms and may optionally have functional groups as substituents,
X : H or an acidic group or a radical having at least 2 OH groups or an amine oxide group,
wherein the compound (I) comprises at least one R⁵ radical of the general formula -CH₂-CH(R^{7a})- (IVa) where R^{7a} is a group selected from the group of - COOR⁸ and -CH₂-O-(-CH₂-CH(R⁹)-O-)₂-R¹⁰, and R⁸, R⁹, R¹⁰ and z are each defined as follows:
R⁸: H, an a-valent ion of the general formula ¹/ₐ M^{a+}, where a = 1, 2 or 3 or a hydrocarbyl group having 1 to 6 carbon atoms,
R⁹: H or a hydrocarbyl group having 1 to 6 carbon atoms,
R¹⁰: H or a hydrocarbyl group having 1 to 6 carbon atoms,
z: a number from 1 to 20.

2. A compound according to claim 1, wherein R^{7a} is a-COOR⁸ group and R⁸ is a methyl and/or ethyl group.

3. A compound according to claim 1, wherein R^{7a} is a-COOR⁸ group and R⁸ is H, an alkali metal ion or an ammonium ion.

4. A compound according to claim 1, wherein R^{7a} is a-CH₂-O-(-CH₂-CH(R⁹)-O-)_{z}-R¹⁰ group where at least 50 mol% of the R⁹ radicals present are H, and R¹⁰ is a methyl or ethyl group and z is a number from 2 to 10.

5. A compound according to any of claims 1 to 4, wherein compounds (I), as well as (IVa) radicals, also comprise -CH₂-CH(R^{7b})- (IVb) radicals where R^{7b} is selected from the group of H, methyl and ethyl.

6. A compound according to claim 5, wherein R^{7b} is H.

7. A compound according to claim 1, wherein the R² radicals are each independently radicals of the general formula -(-CH₂-CH(R^{7b})-O-)ₐ-(-CH₂CH(-COOR⁸)-O-)_{b}-H (V) where the alkylene oxide blocks are arranged in the sequence specified, R^{7b} and R⁸ are each as defined above and a and b are each numbers from 1 to 49, where the sum of a + b is 2 to 50.

8. A compound according to claim 7, wherein a is 2 to 30 and b is 1 to 20 in the formula (V), with the proviso that a > b.

9. A compound according to claim 7 or 8, wherein R⁸ is H, an alkali metal ion or an ammonium ion.

10. A compound according to any of claims 1 to 6, wherein the acidic group is selected from the group of carboxyl groups -COOM, sulfo groups -SO₃M, sulfate groups -OSO₃M, phosphonic acid groups -PO₂M₂ or phosphoric acid groups -OPO₃M₂, where M is H⁺ or a monovalent counterion.

11. A compound according to any of claims 1 to 6, wherein the radical having at least 2 OH groups is a mono- or oligosaccharide radical.

12. A compound according to any of claims 1 to 11, wherein the compounds have the general formula (II) 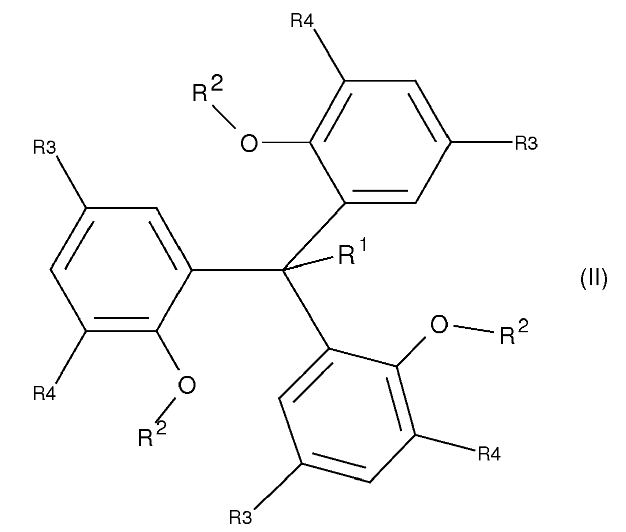 where R¹ and R² are each as defined above and R³ and R⁴ are each independently H or a C₁- to C₃₀-hydrocarbyl radical.

13. A compound according to claim 12, wherein R³ and R⁴ are each independently straight-chain or branched aliphatic C₁- to C₆-hydrocarbyl radicals.

14. A compound according to claim 13, wherein R³ and R⁴ are each t-butyl radicals.

15. A process for preparing the compounds according to any of claims 1 to 14, which comprises providing a starting compound of the general formula (VI) 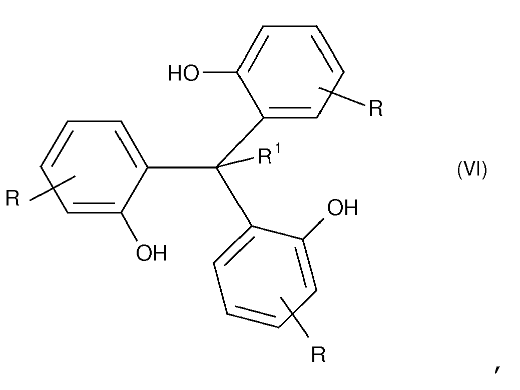 alkoxylating it with alkylene oxides of the general formulae 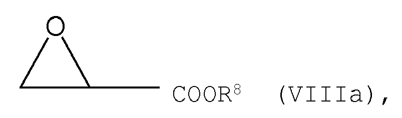 where R⁸ is a hydrocarbyl group having 1 to 6 carbon atoms, and/or 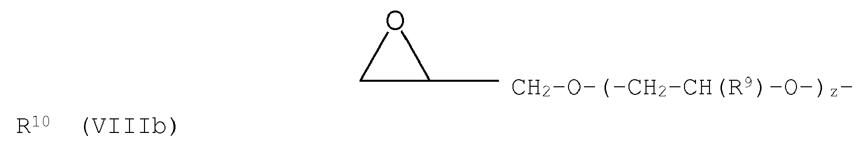 and optionally different C₂- to C₈-alkylene oxides, glycidol and cyclic C₃- to C₉-carbonates, and optionally substituting at least some terminal hydrogen atoms thereon for -R⁶-X radicals, where Z, R, R¹, R⁶, R⁸, R⁹ and R¹⁰ correspond to the definitions in claim 1.

16. The process according to claim 15, wherein alkylene oxides of the formula (VIIIa) are used and COOR⁸ groups present, after the alkoxylation, are hydrolyzed in a further process step to give-COOH groups or salts thereof.

17. The use of compounds according to any of claims 1 to 14 as surfactants and thickeners.

18. The use of compounds according to any of claims 1 to 14 for production of washing and cleaning compositions, dyes and paints, cosmetic and pharmaceutical formulations, paper, textile and leather assistants, formulations for human and animal nutrition, the construction sector, crop protection formulations, and generally for production of emulsions and dispersions.

19. The use of compounds according to any of claims 1 to 14 in processes for mineral oil production.

20. The use according to claim 19, in which an aqueous formulation comprising at least one derivative of tris(2-hydroxyphenyl)methane of the general formula (I) is injected into a mineral oil deposit through at least one injection well and the crude oil is withdrawn from the deposit through at least one production well.

21. The use according to claim 20, wherein the temperature of the mineral oil deposit is 10 to 150°C.

22. The use according to claim 19 or 20, wherein the aqueous formulation comprises, as a further component, at least one salt in an amount of 10 000 ppm to 350 000 ppm.

23. The use according to claim 19, wherein the mineral oil production is effected from deposits with a deposit temperature of 10 to 150°C, said deposit comprising, as well as mineral oil, deposit water with a salinity of 20 000 ppm to 350 000 ppm, and the mineral oil having a viscosity (measured at deposit temperature) of at least 3 mPa*s, by injecting an aqueous formulation comprising at least one derivative of tris(2-hydroxyphenyl)methane of the formula (I) into the mineral oil deposit through at least one injection well and withdrawing crude oil from the deposit through at least one production well, said process comprising at least the following process steps:
- providing at least one tris(2-hydroxyphenyl)methane derivative of the general formula (I) as a pure substance, mixture or concentrate;
- preparing the aqueous formulation of the tris(2-hydroxyphenyl)methane component(s) by diluting the concentrate (K) provided in step (1) on site with water to a concentration of 0.01 g/l to 10 g/l, preferably of 0.05 g/l to 5 g/l,
- injecting the aqueous formulation of the tris(2-hydroxyphenyl)methane component(s) into the mineral oil formation, and
- withdrawing crude oil through at least one production well.

24. The use according to any of claims 19 to 23, wherein the derivative of tris(2-hydroxyphenyl)-methane used is a derivative with unbranched R² radicals and the concentration of the tris(2-hydroxyphenyl)methane derivative in the formulation is 0.01 g/l to 5 g/l.

## Revendications

1. Dérivés de tris(2-hydroxyphényl)méthane de formule générale (I) 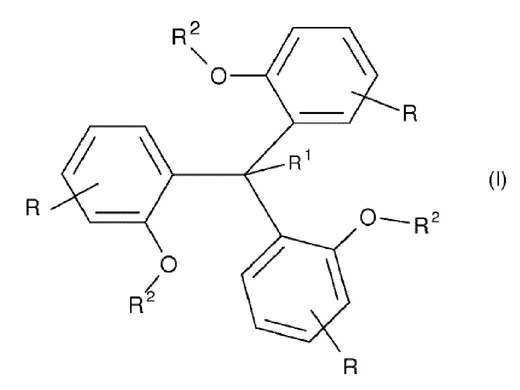 dans laquelle les radicaux R¹, R² et R ont la signification suivante :
R : indépendamment les uns des autres, 0 à 4 radicaux hydrocarbonés en C₁ à C₃₀ par cycle phényle,
R¹ : un radical choisi dans le groupe constitué par H, OH, F, Cl, Br, I, ainsi que les groupes hydrocarbonés en C₁ à C₃₀,
R² : indépendamment les uns des autres, radicaux de formule générale -(-R⁵-O-)ₙ-R⁶-X (III), dans laquelle R⁵, R⁶, X, m et n ont indépendamment les uns des autres la signification suivante :
n : un nombre de 1 à 50,
R⁵ : indépendamment les uns des autres, groupes de formule générale -CH₂-CH(R⁷)-(-CH₂)ₘ- (VI), dans laquelle m représente 0 ou 1 et R⁷ représente un radical choisi dans le groupe constitué par H, les groupes hydrocarbonés en C₁ à C₆, ainsi que les groupes fonctionnels contenant de l'oxygène,
R⁶: une simple liaison ou un groupe alkylène de 1 à 10 atomes de carbone, qui peut éventuellement comprendre des groupes fonctionnels en tant que substituants,
X : H ou un groupe acide ou un radical contenant au moins 2 groupes OH ou un groupe aminoxyde, **caractérisés en ce que** le composé (I) comprend au moins un radical R⁵ de formule générale -CH₂-CH(R^{7a})- (IVa), dans laquelle R^{7a} représente un groupe choisi dans le groupe constitué par -COOR⁸ et -CH₂-O-(-CH₂-CH(R⁹)-O-)_{z}-R¹⁰ et R⁸, R⁹, R¹⁰ et z ont la signification suivante :
R⁸ : H, un ion a-valent de formule générale ¹/ₐ M^{a+}, avec a = 1, 2 ou 3, ou un groupe hydrocarboné de 1 à 6 atomes de carbone,
R⁹ : H ou un groupe hydrocarboné de 1 à 6 atomes de carbone,
R¹⁰ : H ou un groupe hydrocarboné de 1 à 6 atomes de carbone,
z : un nombre de 1 à 20.

2. Composés selon la revendication 1, **caractérisés en ce que** R^{7a} consiste en un groupe -COOR⁸ et R⁸ représente un groupe méthyle et/ou éthyle.

3. Composés selon la revendication 1, **caractérisés en ce que** R^{7a} consiste en un groupe -COOR⁸ et R⁸ représente H, un ion de métal alcalin ou un ion ammonium.

4. Composés selon la revendication 1, **caractérisés en ce que** R^{7a} consiste en un groupe -CH₂-O-(-CH₂-CH(R⁹)-O-)_{z}-R¹⁰, au moins 50 % en moles des radicaux R⁹ présents représentant H, et R¹⁰ consistant en un groupe méthyle ou éthyle, et z consistant en un nombre de 2 à 10.

5. Composés selon l'une quelconque des revendications 1 à 4, **caractérisés en ce que** les composés (I) comprennent en plus des radicaux (IVa) également des radicaux -CH₂-CH(R^{7b})- (IVb), R^{7b} étant choisi dans le groupe constitué par H, méthyle et éthyle.

6. Composés selon la revendication 5, **caractérisés en ce que** R^{7b} représente H.

7. Composés selon la revendication 1, **caractérisés en ce que** les radicaux R² consistent indépendamment les uns des autres en des radicaux de formule générale -(-CH₂-CH(R^{7b})-O-)ₐ-(-CH₂CH(-COOR⁸)-O-)_{b}-H (V), les séquences oxyde d'alkylène étant agencées dans l'ordre indiqué, R^{7b} et R⁸ ayant la signification définie précédemment, et a et b représentant chacun des nombres de 1 à 49, la somme a + b étant de 2 à 50.

8. Composés selon la revendication 7, **caractérisés en ce que**, dans la formule (V), a représente 2 à 30, b représente 1 à 20, à condition que a > b.

9. Composés selon la revendication 7 ou 8, **caractérisés en ce que** R⁸ représente H, un ion de métal alcalin ou un ion ammonium.

10. Composés selon l'une quelconque des revendications 1 à 6, **caractérisés en ce que** le groupe acide est choisi dans le groupe constitué par les groupes carboxyle -COOM, les groupes acide sulfonique - SO₃M, les groupes sulfate -OSO₃M, les groupes acide phosphonique -PO₂M₂ ou les groupes acide phosphorique-OPO₃M₂, M représentant H⁺ ou un contre-ion monovalent.

11. Composés selon l'une quelconque des revendications 1 à 6, **caractérisés en ce que** le radical contenant au moins 2 groupes OH est un radical mono- ou oligosaccharide.

12. Composés selon l'une quelconque des revendications 1 à 11, **caractérisés en ce que** les composés présentent la formule générale (II) 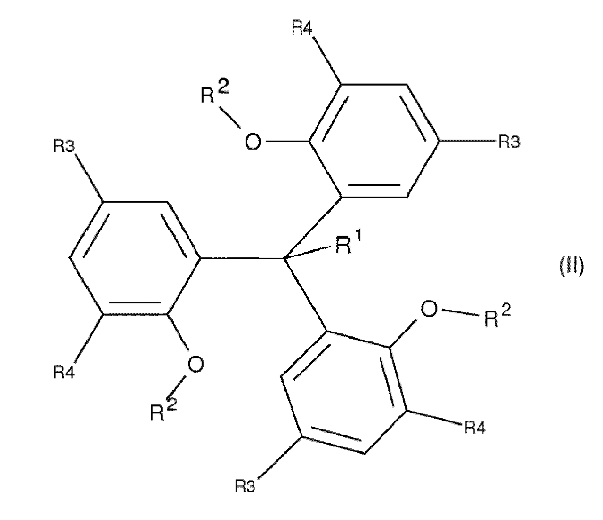 dans laquelle R¹ et R² ont la signification indiquée précédemment, et R³ et R⁴ représentent indépendamment l'un de l'autre H ou un radical hydrocarboné en C₁ à C₃₀.

13. Composés selon la revendication 12, **caractérisés en ce que** R³ et R⁴ représentent indépendamment l'un de l'autre des radicaux hydrocarbonés en C₁ à C₆ aliphatiques linéaires ou ramifiés.

14. Composés selon la revendication 13, **caractérisés en ce que** R³ et R⁴ représentent chacun des radicaux t-butyle.

15. Procédé de fabrication des composés selon l'une quelconque des revendications 1 à 14, **caractérisé en ce qu'**un composé de départ de formule générale (VI) 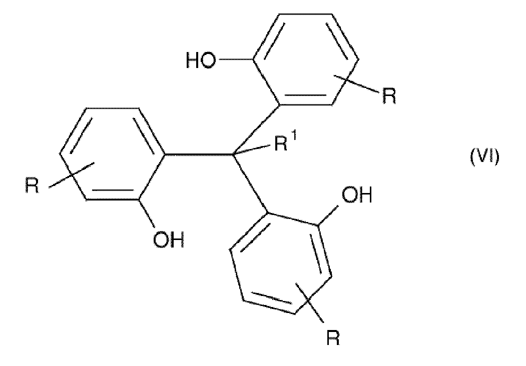 est préparé, alcoxylé avec des oxydes d'alkylène de formule générale 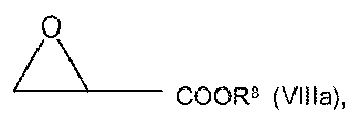 dans laquelle R⁸ représente un groupe hydrocarboné de 1 à 6 atomes de carbone, et/ou 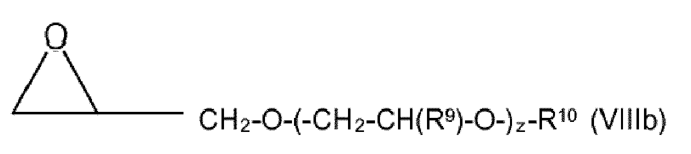 ainsi qu'éventuellement des oxydes d'alkylène en C₂ à C₈ différents de ceux-ci, du glycidol et des carbonates en C₃ à C₉ cycliques, et les atomes H terminaux sont éventuellement remplacés au moins en partie par des radicaux -R⁶-X, z, R, R¹, R⁶, R⁸, R⁹ et R¹⁰ ayant les définitions indiquées dans la revendication 1.

16. Procédé selon la revendication 15, **caractérisé en ce que** des oxydes d'alkylène de formule (VIIIa) sont utilisés et les groupes COOR⁸ présents sont hydrolysés après l'alcoxylation lors d'une étape de procédé supplémentaire en groupes -COOH ou leurs sels.

17. Utilisation de composés selon l'une quelconque des revendications 1 à 14 en tant que tensioactifs et épaississants.

18. Utilisation de composés selon l'une quelconque des revendications 1 à 14 pour la préparation de détergents, de colorants et de peintures, de formulations cosmétiques et pharmaceutiques, d'adjuvants pour papier, textile et cuir, de formulations pour l'alimentation humaine et animale, pour le secteur du bâtiment, de formulations phytosanitaires, ainsi que de manière générale pour la préparation d'émulsions et de dispersions.

19. Utilisation de composés selon l'une quelconque des revendications 1 à 14 dans des procédés d'extraction de pétrole.

20. Utilisation selon la revendication 19, dans laquelle une formulation aqueuse, qui contient au moins un dérivé de tris(2-hydroxyphényl)méthane de formule générale (I), est injectée dans un gisement de pétrole par au moins un forage d'injection et le pétrole brut est extrait du gisement par au moins un forage de production.

21. Utilisation selon la revendication 20, **caractérisée en ce que** la température du gisement de pétrole est de 10 à 150 °C.

22. Utilisation selon la revendication 19 ou 20, **caractérisée en ce que** la formulation aqueuse contient en tant que composant supplémentaire au moins un sel en une quantité de 10 000 ppm à 350 000 ppm.

23. Utilisation selon la revendication 19, **caractérisée en ce que** l'extraction de pétrole a lieu à partir de gisements ayant une température de gisement de 10 à 150 °C, le gisement comprenant en plus du pétrole de l'eau de gisement ayant une salinité de 20 000 ppm à 350 000 ppm, et le pétrole présentant une viscosité (mesurée à la température du gisement) d'au moins 3 mPa*s, une formulation aqueuse comprenant au moins un dérivé de tris(2-hydroxyphényl)méthane de formule (I), étant injecté dans le gisement de pétrole par au moins un forage d'injection et le pétrole brut étant extrait du gisement par au moins un forage de production, le procédé comprenant au moins les étapes de procédé suivantes :
- la préparation d'au moins un dérivé de tris(2-hydroxyphényl)méthane de formule générale (I) sous la forme d'une substance pure, d'un mélange ou d'un concentré ;
- la fabrication de la formulation aqueuse du ou des composants tris(2-hydroxyphényl)méthane par dilution du concentré (K) préparé selon l'étape (1) sur place avec de l'eau à une concentration de 0,01 g/l à 10 g/l, de préférence de 0,05 g/l à 5 g/l,
- l'injection de la formulation aqueuse du ou des composants tris(2-hydroxyphényl)méthane dans la formation pétrolifère, et
- l'extraction de pétrole brut par au moins un forage de production.

24. Utilisation selon l'une quelconque des revendications 19 à 23, **caractérisée en ce que** le dérivé de tris(2-hydroxyphényl)méthane utilisé est un dérivé contenant des radicaux R² non ramifiés et la concentration du dérivé de tris(2-hydroxyphényl)méthane dans la formulation est de 0,01 g/l à 5 g/l.
